# EUROPEAN PATENT APPLICATION

(11) **EP 3 369 595 A1**
(43) Date of publication of application: **05.09.2018**
(21) Application number: 16860371.0
(22) Date of filing: 24.10.2016
(51) Int. Cl.: B60H 3/00, B61D 27/00, A61L 9/20

(54) **RAILWAY CAR AIR CONDITIONING SYSTEM**

(30) Priority: 28.10.2015 RU 2015146102
(71) Applicant: Kiknadze Nina Nikolaevna, Moscow, 119019 (RU)
(72) Inventor: KIKNADZE, Nikolai Jemalovich, Moskow, 119019 (RU)
(74) Representative: Sloboshanin, Sergej
(86) International application number: PCT/RU2016/000727
(87) International publication number: WO 2017/074226

(57) **Abstract**

The invention relates to air conditioning in railway cars. The present air conditioning system comprises an inlet recirculating air duct (1) and an inlet air duct (2) for external air, which are connected to a chamber (3) for mixing external air and recirculated air. Downstream of the mixing chamber (3) is a main air duct (4), in which an evaporation unit (5) and an inlet fan (6) are fastened. The inlet fan is mounted downstream of the evaporation unit, as is an outlet forced-draft air duct (7) for supplying treated air to the passenger compartment. The system is provided with an ultraviolet air treatment unit (8), in which primarily U-shaped amalgam lamps (13) are mounted as a source of UV radiation. Unit (8) can be mounted in one or several positions in the system. The inside surface of the unit (8) can have a coating applied thereto, which absorbs or reflects ultraviolet radiation. The technical result is a system which provides more effective decontamination of air, remains fit for use for longer, has a longer service life, protects people from UV radiation, has smaller dimensions and is easier to install and maintain.

## Description

### Field of the invention

The invention relates to transport engineering, in particular to air-conditioning installations intended for ventilation and air conditioning systems for passenger compartments, in particular for railway carriages, as well as other kinds of public transport, and can be used as a device for decontaminating air entering a passenger compartment by means of germicidal ultraviolet (UV) radiation for ensuring epidemiological environmental safety in means of transport.

### Prior art

In accordance with to health requirements, recirculated air contaminated by human beings, which is collected from passenger compartments and returned to the device, namely the air conditioning system (hereinafter the ACS), is subject to mandatory decontamination with the objective of preventing the spread of infections and bacterial infection in passenger trains. The main source of air contamination by micro-organisms, including pathogens, is people. On average, each person emits several thousands of micro-organisms per hour into the surrounding air (when coughing, up to several tens of thousands). The microorganisms emitted into the air spread in the form of tiny droplets of moisture containing the microorganisms, a significant quantity of which settles on surfaces, and therefore after the evaporation of the moisture, enters the air.

The problem of reducing the number of microorganisms in the air, and eliminating the spread of infectious diseases through ventilation and air conditioning systems installed in passenger compartments of public transport vehicles is solved by the use of germicidal UV radiation.

Various devices for the disinfection of air are known from the prior art: CA 2879137 A1; CN 1772309 A; CN 2683130 Y; CN 201181064 Y; CN 201437322 U; RU 2340360 C2; RU 2416432 C1; RU 2506501 C1; RU 2008119428 (WO 2007045729 A1); SU 1210839 A1; SU 1351607 A1; TW 200422566 A; US 5505904 A; US 5817276 A; US 5894130 A; US 6438971 B1; US 2003099569 A1; US 2008053311 A1; US 2008152548 A1; US 2009123343 A1; US 2010041328 A1; US 2012076700 A1.

In particular, an air conditioning apparatus for a passenger railway car is known, which consists of a compression-type refrigerating machine, air cleaning filters, devices for supplying the external and recirculated air, regulating valves, chambers for mixing the external and recirculated air flow, devices for dispersing the air from the forced-draft air duct in passenger compartments, devices for removing the air from the car, and an electrical power supply. In channels of the air tract of a ventilation system, ultraviolet (UV) radiation disinfecting devices are installed, which are connected to the electrical power supply. Decontamination devices may be installed in either the recirculated air stream between the cleaning filter up and the air inlet into the air cooler or fan, or in the external air stream between the cleaning filter and the air inlet into the air cooler or fan, or installed in the stream of the mixture of external and recirculated air between the cleaning filter and the air inlet into the air cooler or fan, or installed in the forced-draft air duct before the devices for air distribution into the passenger compartments, or in all of the places listed above at the same time (RF Patent #2278794, B61D 27/00, 2004).

The air decontamination devices used are light sources which are formed by electric discharge arcs without the use of mercury vapor, for example, a matrix of open electrical discharges or a single open electrical arc.

A disadvantage of the known technical solution is the lack of means for protecting the constituent components of the ACS (filters, parts of the fan, etc.) from the damaging effects of UV radiation, as the decontamination devices are installed directly in the channels of the air ducts, which greatly reduces the service life of the railway car. In addition, ensuring the desired effectiveness of the disinfection requires high energy consumption to generate the arc discharge that provides the standard dose of UV radiation. Installation and operation of such devices in the air ducts is complex and labor intensive, and requires significant additional space in the ACS of the car which impairs the operational properties of the ACS.

A ventilation device in an ACS for a passenger car is known, comprising serially connected inlet gills for the external air, mixing chambers, air filters, a fan, an air heater and a forced-draft air duct with outlets into a compartment of the car, a recirculated air duct with inlet collecting grilles, which connects the inlets and the outlets of the mixing chambers, and germicidal UV lamps installed on the cover flap of the air duct on its inlet downstream of the collecting grilles, and separated from the recirculated air duct by a transparent screen made of quartz or uviol glass, for focusing the UV radiation. On the internal wall of the recirculated air duct, screens are arranged which reflect the UV radiation multiple times (RF utility model patent #29512, B61D 27/00, 2002).

A disadvantage of the known utility model patent is that the elements of the air recirculation duct are not protected from the damaging effects of direct and reflected UV radiation, and its lack of convenience in terms of installation and operation, since the UV lamps are suspended from the cover flap.

A system is known for decontamination ventilated air using ultraviolet lamps, which contains an air conditioning unit consisting of two exhaust fans, a compressor, an electrical distribution box with compartments for high and low voltages, and of a system of circulation channels including a ceiling channel for the inlet air, drainage and floor channels, a CO2 level sensor and at least two ultraviolet lamps, which are mounted directly in the air mixing area upstream of the filters to allow the exposure of a maximum number of simple-structured micro-organisms to ultraviolet radiation and thereby neutralize them. In this arrangement the ultraviolet lamps are arranged in such a way that their radiations partially overlap (Russian Federation utility model patent #134879, 2013, B61D 27/00).

The utility model patent is aimed at ensuring the epidemiological safety of recirculated air and its effective decontamination. UV lamps are arranged directly upstream of the air mixture filter, which is one of the most significant places in the growth of bacteria, and are arranged in such a way as to ensure the optimum irradiation and decontamination of the filter. To ensure a longer service life the UV lamps are only turned on in the normal operating mode of the the air-conditioning unit.

A disadvantage of this prior-art solution is the absence of a means for protecting the assemblies of the air-conditioning system, made from materials which are unstable under the effects of UV radiation, from the effects of direct and reflected UV radiation, and also the possibility of the escape of UV radiation through the filter into the passenger compartment.

The closest analogue from the prior art is taken as being the technical solution according to international application WO 2004065148 A2 "System and method for the treatment of micro-organisms inhabiting heating, ventilation, and air conditioning system of vehicles".

The international application WO 2004065148 describes a "System and method for the treatment of micro-organisms inhabiting heating, ventilation, and air conditioning system of vehicles". The invention according to WO 2004065148 relates to heating, ventilation and air conditioning (HVAC) systems for vehicles, and more specifically to a system and a method for reducing the quantity of micro-organisms in ventilation and air conditioning systems. The ventilation and air conditioning system contains an external air intake and a recirculated air intake for collecting the air from the passenger compartment of the vehicle. Air intake units provide a supply of air collected from the area outside of the vehicle and air from the passenger compartment (air recirculation), respectively, and the system also comprises sources of UV radiation. The surfaces or coating materials of the assemblies and elements of the system in the channels and pipes between the ultraviolet radiation source and the passenger compartment may be implemented such that they absorb (not reflecting UV radiation) and reflect UV radiation. According to a first design variant of the invention, the sources of ultraviolet radiation are arranged in the channels of the external air intake unit and the compartment air intake unit of the vehicle and, in particular, also above the drainage sump of the system. The sources of UV radiation provide UV treatment of the air, the air intake units and the surrounding channels of the systems, wherein all the air at the inlet to the system is exposed to the action of UV light. According to a second implementation variant of the invention the UV radiation source is located in the pipework between the external and recirculated air intake units and the fan of the ventilation and air-conditioning system, to ensure that all air at the inlet into the system and the adjacent elements of the system are exposed to the action of the UV radiation source. According to a third implementation variant of the invention the UV radiation source is located between the fan and the evaporation unit of the ventilation and air conditioning system, so that the air passing through the pipework downstream of the fan, and the surrounding elements of the system, can be exposed to the UV radiation. According to a fourth implementation variant of the invention the UV radiation source is located in the evaporation unit of the ventilation and air conditioning system, to treat the heat-exchange surfaces of the evaporation unit and the surrounding components of the system, such as the sump etc. When the heat exchange surfaces are exposed to UV radiation, these surfaces are less susceptible to the accumulation of micro-organisms (formation of a biofilm), which allows the efficiency of the heat transfer to be increased due to the absence of a biofilm. According to a fifth implementation variant of the invention the UV radiation source is located between the evaporation unit and the heating unit of the ventilation and air conditioning system, which allows all the air passing into the section downstream of the evaporation unit to be processed. In addition, the adjacent elements of the system, such as the air ducts, are also exposed to the action of the UV light. According to a sixth variant of the invention the UV radiation source is located next to the heating unit of the HVAC system, to ensure that irradiation of the heat exchange surfaces of the heater components and the surrounding components of the system. When the heat exchange surfaces are exposed to ultraviolet radiation, the surfaces will be less inclined to the accumulation of micro-organisms and formation of biofilms, which allows the efficiency of the heat exchange to be increased. According to a seventh version, the UV radiation source is arranged between the heating unit and the outlet channel, oriented downwards. The positioning of the UV radiation source at this location ensures that all the air passing through into the parts of the system downstream of the heater is exposed to UV radiation. In addition, the ventilation and air conditioning components, including parts of the heater and the air ducts/pipes are also exposed to ultraviolet radiation. According to an eighth version of the invention, the UV radiation sources are arranged in the outlet channels of the ventilation and air conditioning system. The arrangement of the sources of ultraviolet radiation in these locations ensures that all air at the outlet from the system is exposed to UV light radiation. In addition, the ventilation and air conditioning components, including parts of the heater and the air ducts/pipes are also exposed to irradiation by ultraviolet light.

In the closest equivalent solution the different layout schemes of the UV radiation sources are described in detail, wherein mention is made of the materials of the coatings used for absorbing or reflecting UV radiation. However, no concrete implementation of components of the system is provided for allowing the protection of passengers and staff as well as the assemblies and components of the system against the effects of UV radiation, and at the same time increasing the effectiveness of the air treatment, reducing the dimensions of the system and ensuring its high performance.

### Description of the invention

The object (technical problem) of the disclosed invention is to improve the operational characteristics of an ACS by improving the reliability of the protection from UV exposure of people (passengers, staff) and of the components of ventilation and air conditioning devices, as well as reducing the dimensions of the system while maintaining high performance. The necessity to achieve this object is due to the fact that some of the assemblies and components of ventilation and air conditioning systems are manufactured from materials which are unstable under the effects of UV radiation, as a result of which with increasing exposure time and intensity of the UV radiation, an ageing effect of polymer materials becomes apparent along with a loss of durability of the construction, which leads to the subsequent gradual destruction of the assemblies and components of the device. UV radiation exposure in human beings can cause burns to the eyes, the skin of the face, hands and other uncovered parts of the body, which are unacceptable. Decontaminated air, having passed through the ACS, is supplied to the compartment continuously, where it is mixed with a volume of air infected by microbiological contaminants emitted by the people occupying the space. To guarantee the disinfection of the air requires the use of sufficiently powerful sources of UV radiation, to ensure the disinfection of air at high flow rates. The air flow rate determines the performance of the ACS (the "recirculation ratio" - the amount of decontaminated air per unit of time in relation to the total volume of the interior of the vehicle compartment), that is necessary to obtain a high degree of purification of the air from micro-organisms.

The technical result achieved by the implementation of the invention includes increasing the efficiency of the disinfection of air while increasing performance (a recirculation ratio of 2-27, which provides sufficient reduction of the growth of the colony of micro-organisms) while extending the service life and fitness for use of the ACS (protection of assemblies and parts from exposure to UV light), as well as protecting passengers and staff from UV radiation, in addition to a reduction in the size of the system and simplifying its installation and maintenance.

To ensure the protection of the vehicle passengers against pathogenic micro-organisms, as noted above, it is necessary to ensure decontamination of a large volume of air per unit of time, wherein the dimensions in which the air conditioning system is to be installed are determined by the design of the railway car or other passenger vehicle, which imposes limits on the dimensions of the system and the dimensions of the UV lamps used. In addition, when using high-power UV lamps, the assemblies and components of the recirculated air duct are subject to significant destructive effects of direct and reflected UV radiation, and the escape of UV radiation outside of the confines of the air conditioning system (from either the input or output opening) can cause harm to passengers. To achieve the results indicated above, it is necessary to satisfy the following mutually exclusive conditions: the air conditioning system must ensure a high degree of disinfection of air (to destroy micro-organisms that are mainly present in the recirculated air stream) and high system performance, wherein it also necessary to prevent the release of UV radiation outside the system (to preclude any impact on human beings) and to minimize the impact of UV radiation on the assemblies and components of the ACS, the material of which is damaged under prolonged exposure to UV radiation, and the system must additionally have small dimensions and be convenient to install and maintain.

A railway car ACS, such as the system from the nearest prior art solution, comprises a mixing chamber, which external and recirculated air enters via corresponding inlet ducts, a main air duct, in which are installed an evaporation unit and inlet fan, an output forced-draft air duct supplying air into the compartment, and the UV radiation sources, which can be installed upstream of the mixing chamber and/or in the mixing chamber and/or downstream of the mixing chamber upstream of the evaporation unit and/or downstream of the inlet fan, wherein the system can be equipped with means for protecting against the release of UV radiation, and in addition a coating may be used that absorbs UV radiation, as well as a coating that reflects UV radiation.

The characterizing features of the claimed invention are a different configuration of the system - the evaporation unit is installed upstream of the inlet fan, which allows the energy consumption in the circulation of the air flow to be reduced, the cooling performance to be increased and the dimensions of the device to be reduced. In this arrangement of the inlet fan air flows evenly through the evaporation unit. If the fan is arranged upstream of the evaporator, the energy consumption will increase, the non-uniform flow leads to local frosting of the evaporator, to uneven processes of evaporation in the evaporator and, as a consequence, to a reduction in the cooling performance, wherein in order to equalize the flow it will be necessary to increase the distance from the evaporator, which will also increase the size of the device.

In addition, the ACS is equipped with an ultraviolet air treatment unit (UAT unit), in which a UV radiation source is arranged, in which the UAT unit consists of an air-duct housing having windows for inlet and outlet of the treated air, which is part of the general air channel of the ACS, and lamp assemblies with electrical connectors for one, two or more sources of germicidal UV radiation are secured to the wall of the air-duct housing of the UV air treatment unit, in which U-shaped amalgam lamps are used as the sources of germicidal UV radiation. The use of a removable UAT unit to accommodate the UV radiation source in the described design allows the unit to be integrated into the ACS at the desired location, creating the conditions for reliable protection of both people and the assemblies and components of the system from the effects of UV radiation, and allows the size to be minimized and the ease of installation and maintenance improved. The choice of amalgam lamps as a source of germicidal UV radiation provides improved energy efficiency of the system and a high level of disinfection for a broad range of micro-organisms including viruses, which allows high performance of the ACS and a reduction of its dimensions.

If the UAT unit is arranged upstream of the evaporation unit, or downstream of it and upstream of the inlet fan, the UAT unit can be mounted coaxially with the main air duct and rigidly connected to it.

If the UAT unit is arranged near to the inlet window for the recirculated air or in the recirculation air duct or downstream of the inlet fan in the forced-draft air duct before the outlet window of the treated air, then the UAT can be mounted in accordance with a configuration of the ACS, in which the UAT unit is rigidly connected to the recirculated air duct or the forced-draft air duct respectively.

If more than one amalgam lamp or lamp assembly is used, for example for two amalgam lamps, they are mounted in pairs one under another or along the axis of the air-duct housing of the unit on a single line, or with a height offset to allow the arrangement of the amalgam lamps one under the other or sequentially one after the other (in a straight line or offset in height) for efficient treatment of the air and to provide a means of varying the dimensions of the UAT unit along the length and height, depending on the mounting position of the system.

To provide protection for human beings as well as assemblies and parts of the system from the effects of UV radiation depending on the mounting position of the UAT unit, means for protecting against the release of UV radiation, in the form of protective profiled grilles, are mounted on the inlet and/or outlet windows of the air-duct housing. A photocatalytic coating based on titanium dioxide is applied to the surface of the protective profiled grilles, which enables a process of photocatalysis to be optionally used to enhance the effectiveness of the disinfection and cleaning of the air. A titanium-dioxide based photocatalyst is usually applied to the surface of the protective grille and/or on the internal surface of the UAT unit by means of spraying. In the process of photocatalysis the titanium dioxide, which absorbs ultraviolet light, produces free radicals, which effectively oxidize organic material, including viruses, bacteria and other micro-organisms and, in addition, volatile organic compounds decompose into harmless water molecules and carbon dioxide gas.

In addition to the coating which absorbs UV radiation, when materials that are resistant to UV radiation are used, the UAT unit can be coated on the internal surface with a coating that reflects UV radiation. The use of a reflective coating is aimed at expanding the region of continuous exposure of the processed air to UV radiation in places remote from the radiation source and located outside the confines of the UAT unit. In this case, the distribution of the action of the ultraviolet radiation over the air flow outside the UAT unit increases the probability of almost total elimination of the living micro-organisms.

It is also possible to use combinations of coatings that absorb UV radiation and reflect UV radiation.

Depending on the mounting point of the UAT unit and the materials used in the ACS, either resistant or non-resistant to UV radiation, the coating that absorbs the UV radiation (titanium dioxide), and the coating reflecting the UV radiation are applied to different parts of the UAT unit.

The desired implementation variant of the UAT unit and place of application of the coating for absorbing and/or reflecting the UV radiation are selected depending on the specific situation, including the materials used in the construction of parts of the system.

If the UAT unit is installed upstream of the mixing chamber from the direction of the recirculated air inlet, the protective grilles are installed on both windows of the UAT unit, i.e. on the inlet window for the recirculated air and the outlet window for the air into the mixing chamber. A coating of titanium dioxide is applied on the internal surface of the air-duct housing of the UAT unit, the titanium dioxide coating also being applied to both grilles.

As a design variant, the protective grille with a titanium dioxide coating is only installed on the inlet window for the recirculated air, and the second grille on the air outlet window is omitted. The titanium dioxide coating, as in the version described above, is applied to the internal surface of the air-duct housing of the UAT unit. Almost no release of UV radiation in the direction of the mixing chamber occurs, since the UV radiation is absorbed by the coating on the internal surface of the UAT unit.

If the UAT unit is arranged directly in the mixing chamber, the following design variants for the UAT unit are possible.

A first design variant with the UAT unit mounted in the mixing chamber: the protective grilles can be installed on both windows of the UAT unit, i.e. on the air inlet window into the UAT unit and on the outlet window for the air into the mixing chamber. The coating of titanium dioxide is applied on the internal surface of the air-duct housing of the UAT unit, the titanium dioxide coating also being applied to both grilles, thereby preventing the release of UV radiation beyond the confines of the UAT unit, protecting the assemblies and parts of the system from the effect of UV radiation.

A second design variant with the UAT unit mounted in the mixing chamber: the protective grille with the titanium dioxide coating can be installed only on the inlet window for the recirculated air into the UAT unit, and the second grille on the air outlet window is omitted. The titanium dioxide coating, as in the version described above, is applied to the internal surface of the air-duct housing of the UAT unit.

A third design variant with the UAT unit mounted in the mixing chamber: the protective grille with the titanium dioxide coating can be installed only on the outlet window for the recirculated air, and the grille on the air inlet window into the UAT unit is omitted. The titanium dioxide coating, as in the version described above, is applied to the internal surface of the air-duct housing of the UAT unit.

A fourth design variant with the UAT unit mounted in the mixing chamber: the protective grilles may be omitted, while the titanium dioxide coating is applied on the internal surface of the air-duct housing of the UAT unit.

A fifth design variant with the UAT unit mounted in the mixing chamber: the protective grilles may be omitted, while a coating that reflects UV radiation is applied to the internal surface of the UAT unit.

A sixth design variant with the UAT unit mounted in the mixing chamber: the protective grille with the titanium dioxide coating can be installed only on the inlet window for the recirculated air into the UAT unit, while a coating that reflects UV radiation is applied to the internal surface of the UAT unit.

If the UAT unit is mounted directly downstream of the mixing chamber upstream of the evaporation unit, the following design variants for the UAT unit are possible.

A first design variant with the UAT unit mounted downstream of the mixing chamber and upstream of the evaporation unit: the protective grilles can be installed on both windows of the UAT unit, i.e. on the air inlet window into the UAT unit and on the outlet window for the air in the direction of the evaporation unit. The coating of titanium dioxide is applied on the internal surface of the air-duct housing of the UAT unit, the titanium dioxide coating also being applied to both grilles, thereby preventing the release of UV radiation beyond the confines of the UAT unit, protecting the assemblies and parts of the system from the effect of UV radiation.

A second design variant with the UAT unit mounted downstream of the mixing chamber and upstream of the evaporation unit: the protective grille with the titanium dioxide coating can be installed only on the inlet window for the recirculated air into the UAT unit, and the second grille on the air outlet window is omitted. The titanium dioxide coating, as in the version described above, is applied to the internal surface of the air-duct housing of the UAT unit.

A third design variant with the UAT unit mounted in the mixing chamber: the protective grille with the titanium dioxide coating can be installed only on the outlet window for the recirculated air, and the grille on the air inlet window into the UAT unit is omitted. The titanium dioxide coating, as in the version described above, is applied to the internal surface of the air-duct housing of the UAT unit.

A fourth design variant with the UAT unit mounted downstream of the mixing chamber and upstream of the evaporation unit: the protective grilles may be omitted, while the titanium dioxide coating is applied on the internal surface of the air-duct housing of the UAT unit.

A fifth design variant with the UAT unit mounted downstream of the mixing chamber and upstream of the evaporation unit: the protective grilles may be omitted, while a coating that reflects UV radiation is applied to the internal surface of the UAT unit.

A sixth design variant with the UAT unit mounted downstream of the mixing chamber and upstream of the evaporation unit: the protective grille with the titanium dioxide coating can be installed only on the inlet window for the recirculated air into the UAT unit, while a coating that reflects UV radiation is applied to the internal surface of the UAT unit.

A seventh design variant with the UAT unit mounted downstream of the mixing chamber and upstream of the evaporation unit: the protective grille with the titanium dioxide coating can be installed only on the outlet window for the air to be treated out of the UAT unit, while a coating that reflects UV radiation is applied to the internal surface of the UAT unit.

If the UAT unit is mounted directly downstream of the evaporation unit and upstream of the inlet fan, the following design variants for the UAT unit are possible.

A first design variant with the UAT unit mounted downstream of the evaporation unit and upstream of the inlet fan: the protective grilles can be installed on both windows of the UAT unit, i.e. on the air inlet window into the UAT unit and on the air outlet window in the direction of the inlet fan. The coating of titanium dioxide is applied on the internal surface of the air-duct housing of the UAT unit, the titanium dioxide coating also being applied to both grilles, thereby preventing the release of UV radiation beyond the confines of the UAT unit, protecting the assemblies and parts of the system from the effect of UV radiation.

A second design variant with the UAT unit mounted downstream of the evaporation unit and upstream of the inlet fan: the protective grille with the titanium dioxide coating can be installed only on the inlet window for the air to be treated into the UAT unit, and the second grille on the air outlet window is omitted. The titanium dioxide coating, as in the version described above, is applied to the internal surface of the air-duct housing of the UAT unit.

A third design variant with the UAT unit mounted downstream of the evaporation unit and upstream of the inlet fan: the protective grille with the titanium dioxide coating can be installed only on the air outlet window, and the grille on the air inlet window into the UAT unit is omitted. The titanium dioxide coating, as in the version described above, is applied to the internal surface of the air-duct housing of the UAT unit.

A fourth design variant with the UAT unit mounted downstream of the evaporation unit and upstream of the inlet fan: the protective grilles may be omitted, while the titanium dioxide coating is applied on the internal surface of the air-duct housing of the UAT unit.

A fifth design variant with the UAT unit mounted downstream of the evaporation unit and upstream of the inlet fan: the protective grids may be omitted, while a coating that reflects UV radiation is applied to the internal surface of the UAT unit.

A sixth design variant with the UAT unit mounted downstream of the evaporation unit and upstream of the inlet fan: the protective grille with the titanium dioxide coating can be installed only on the inlet window for the recirculated air into the UAT unit, while a coating that reflects UV radiation is applied to the inner surface of the UAT unit.

If the UAT unit is arranged downstream of the inlet fan, the following design variants for the UAT unit are possible.

A first design variant with the UAT unit mounted downstream of the inlet fan: the protective grilles can be installed on both windows of the UAT unit, i.e. on the air inlet window into the UAT unit and on the air outlet window in the direction of the inlet fan. The coating of titanium dioxide is applied on the internal surface of the air-duct housing of the UAT unit, the titanium dioxide coating also being applied to both grilles, thereby preventing the release of UV radiation beyond the confines of the UAT unit, protecting the assemblies and parts of the system from the effect of UV radiation.

A second design variant with the UAT unit mounted downstream of the inlet fan: the protective grille with the titanium dioxide coating can be installed only on the inlet window for the air to be treated into the UAT unit, and the second grille on the air outlet window is omitted. The titanium dioxide coating, as in the version described above, is applied to the internal surface of the air-duct housing of the UAT unit.

Thus, the inclusion of a UAT unit into the structure of the ACS, which is the main air disinfection component due to the amalgam UV lamps and photocatalytic coating based on titanium dioxide and/or the reflective coating, can significantly improve the level of disinfection of the air while increasing the reliability of the protection of passengers and staff from exposure to UV radiation, by selecting the optimum installation position of the UAT unit and the positions of the titanium dioxide coating or the reflective coating.

Energy-efficient amalgam lamps can be arranged in the UAT unit in a highly compact way - one below another or sequentially one after the other (or otherwise), as required.

### Brief description of the drawings

The invention is illustrated by means of drawings which show:

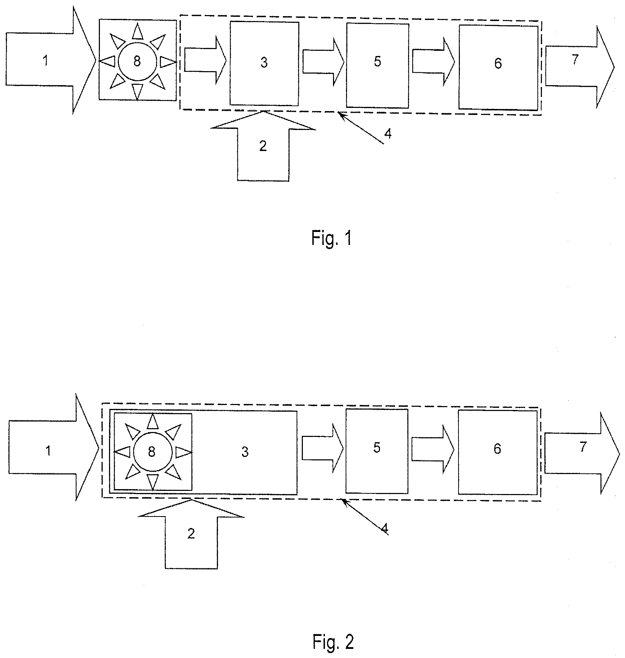

In Fig. 1 - a diagram of an air conditioning system for a railway car compartment with an ultraviolet air treatment unit (UAT) mounted upstream of the mixing chamber downstream of the recirculated air inlet duct.
In Fig. 2 - a diagram of an ACS for a railway car compartment with an ultraviolet air treatment unit (UAT unit) mounted in the mixing chamber.

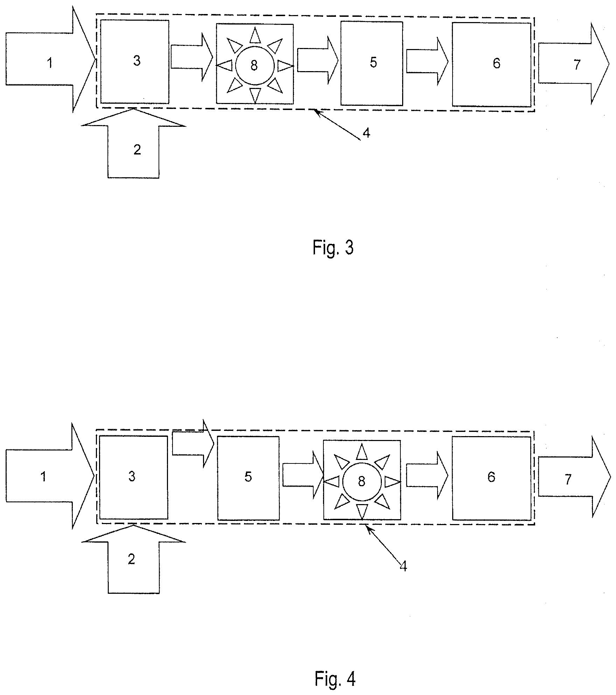

In Fig. 3 - a diagram of an ACS for a railway car compartment with an ultraviolet air treatment unit (UAT unit) mounted downstream of the mixing chamber and upstream of the evaporation unit.
In Fig. 4 - a diagram of an ACS for a railway car compartment with an ultraviolet air treatment unit (UAT unit) mounted downstream of the evaporation unit and upstream of the inlet fan.

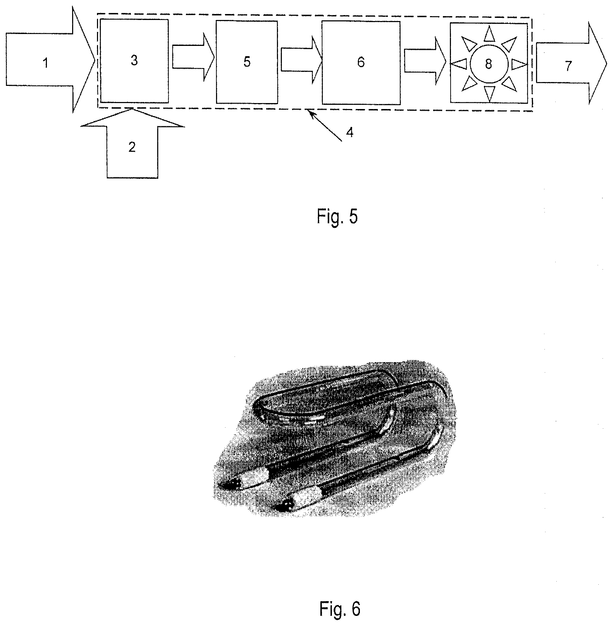

In Fig. 5 - a diagram of an ACS for a railway car compartment with an ultraviolet air treatment unit (UAT unit) mounted downstream of the inlet fan in the forced-draft air inlet duct.
In Fig. 6 - one of the design variants of an amalgam lamp
In Fig. 7, 8 and 9 - example designs of the ultraviolet air treatment unit (UAT unit).
In Fig. 10 - view from below of a design variant of the UAT unit (elements for supplying and controlling amalgam lamps shown).

The ACS for a railway car passenger compartment contains a recirculated air inlet duct 1, an inlet air duct 2 for the external air, both of which are connected to a chamber 3 for mixing the external and the recirculated air. Downstream of the mixing chamber 3 are located the main air duct 4, in which an evaporation unit 5 and an inlet fan 6 are secured, wherein the inlet fan is arranged downstream of the evaporation unit, and the forced-draft air outlet duct 7, which feeds the treated air into the passenger compartment.

In addition, the ACS is equipped with an ultraviolet air treatment unit 8 (UAT), in which a UV radiation source is arranged. The UAT unit 8 consists of an air-duct housing 9 having windows 10 and 11 for the inlet and outlet respectively of the air to be treated. Lamp assemblies 12 with electrical connectors for one, two or more sources of germicidal UV radiation - in the form of amalgam lamps 13, primarily having a U-shaped form - are secured to the wall of the air-duct housing of the UAT unit.

The curved shape of the lamp allows the dimensions of the unit and the system as a whole to be reduced. The UAT unit 8 has the flanges 14 for fixing the unit in a given position in the ACS.

The UAT unit 8 can be installed in the main air duct 4 upstream of the evaporation unit or downstream of it and upstream of the inlet fan, wherein the UAT unit can be mounted coaxially with the main air duct and rigidly connected to it.

If the UAT unit 8 is arranged near to the inlet window for the recirculated air in the inlet recirculated air duct 1 or downstream of the inlet fan 6 in the forced-draft air duct 7 upstream of the outlet window for the treated air, then the UAT unit can be mounted in accordance with a configuration of the ACS, in which the UAT unit is rigidly connected to the recirculated air duct or the forced-draft air duct respectively. In the ACS one, two or more UAT units can be used, which can be positioned at a plurality of locations of the system.

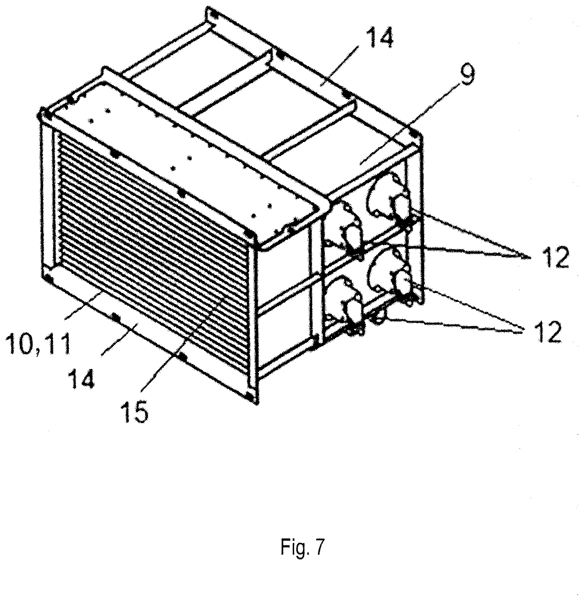

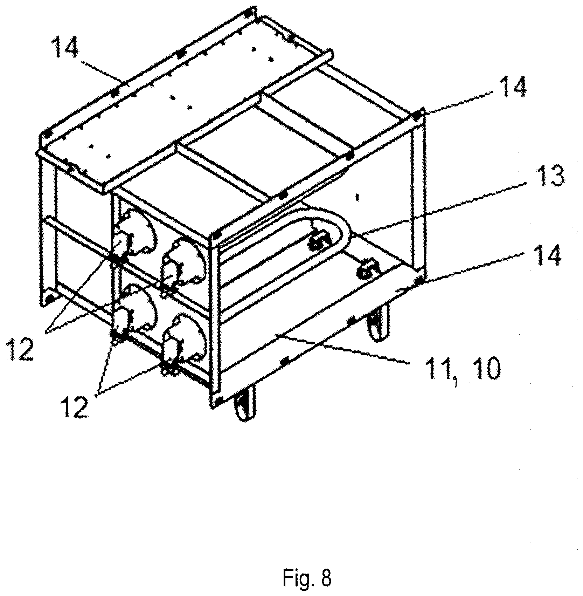

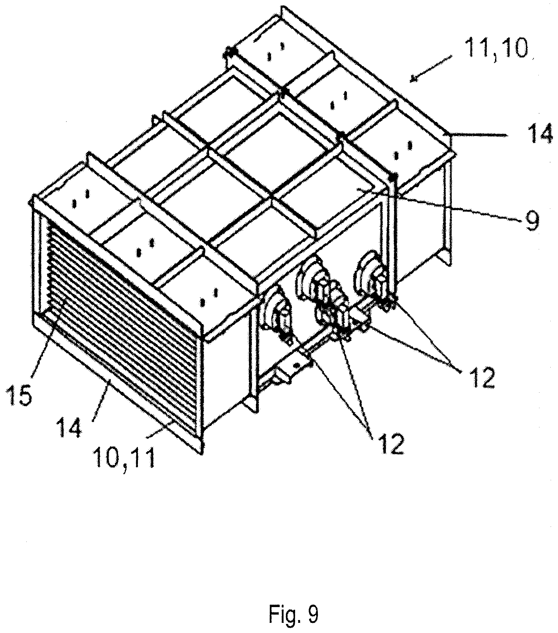

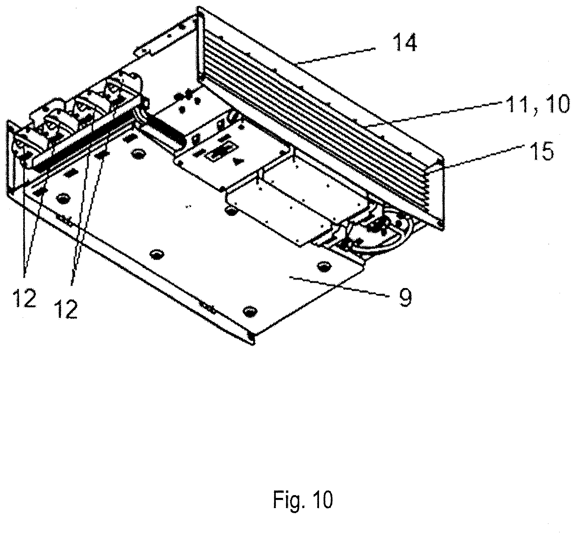

Lamp assemblies 12 for two amalgam lamps are mounted in pairs one below another, as shown in Figs. 7 and 8. Fig. 9 shows the arrangement of the lamp assemblies along the axis of the air duct, with a height offset. It is also possible to arrange the lamp assemblies at the same height - along the axis of the UAT unit. Accordingly, the amalgam lamps 13 can be located one below the other, or sequentially one after the other with a height offset (Fig. 9) or sequentially in a straight line (Fig. 10), which allows intensive air treatment in a unit with a small volume.

On the air inlet window 10 and air outlet window 11 of the air-duct housing 9 of the unit 8, means for protection against UV radiation can be mounted in the form of protective profiled grilles 15. The grilles 15 can be mounted on both windows 10 and 11, on one window 10 or 11 or else completely absent.

The surface of the protective profiled grilles 10 and 11, if they are present, are provided with a photocatalytic coating based on titanium dioxide (TiO₂), that absorbs UV radiation and additionally improves the effectiveness of the disinfection and cleaning of the air.

In addition to the coating that absorbs the UV radiation, if materials are used which are resistant to UV radiation, a coating which reflects the UV radiation can also be applied to the internal surface of the UAT unit 8. This coating ensures the output of UV radiation beyond the confines of the unit 8, which increases the volume which is exposed to the ultraviolet radiation. The invention is also provided with an option to use coatings that reflect UV radiation as well as coatings that absorb UV radiation. The choice of coating and the utility of mounting protective grilles 15 is determined based on the location of the UAT unit 8 and the materials used in the ACS, either resistant or non-resistant to UV radiation.

The desired installation variant of the UAT unit and place of application of the coating for absorbing and/or reflecting the UV radiation are selected depending on the specific situation, including the materials used in the construction of parts of the system, taking into account the fact that, for example, the materials commonly used for isolating the connecting wires, or for the bellows and sleeves of the lamp assemblies 12, are resistant to UV radiation, such as fluoroplastics and the material used for the centering rings for fixing the sockets of the amalgam lamps 13 are normally silicone rubbers. Also resistant to UV radiation is stainless steel, from which the air ducts are manufactured, including the air-duct housing 9 of the UAT unit 8, and ceramic which is used in the sockets for connecting the amalgam UV lamps 13. In addition, materials which are not resistant to the action of UV radiation can also be used, such as galvanized steel, porous rubber, which is used as a sealing material for the maintenance hatch and for other joints.

If the UAT unit is installed upstream of the mixing chamber from the direction of the recirculated air inlet duct 1, the protective grilles 15 are installed on both windows of the UAT unit 8, i.e. on the inlet window 10 for the recirculated air and the air outlet window 11 into the mixing chamber 3. A coating of titanium dioxide is applied to both the protective grilles 15 and, in addition, it may be applied to the internal surface of the air-duct housing 9 of the UAT unit 8. This is to prevent the emission of UV radiation outside the confines of the system through the inlet window for the recirculated air, so that UV radiation does not reach the passengers present in the compartment. The grille 15 on the air outlet window 11 prevents the release of UV radiation in the direction of the mixing chamber 3. The protective grille 15 with the titanium dioxide coating can be installed only on the air inlet window 10, and the second grille on the air outlet window 11 may be omitted, while the internal surface of the air-duct housing of the UAT unit 8 can be coated with titanium dioxide if required, so that it is possible to provide a significant reduction of UV radiation emission in the direction of the compartment, namely on account of the double protection: the UV radiation is absorbed by the coating of the internal surface of the air-duct housing and the protective grille on the air inlet window 10. The emission of UV radiation in the direction of the mixing chamber 3 is suppressed only by the coating on the inside surface of the UAT unit 8. If the materials used in the construction of the mixing chamber 3 are resistant to UV radiation, then it is not necessary to apply a titanium dioxide coating to the internal surface of the air-duct housing 9 of the UAT unit 8.

If the UAT unit 8 is mounted directly in the mixing chamber 3, the protective grilles coated with titanium dioxide can be installed on both windows 10 and 11 of the UAT unit 8. The internal surface of the air-duct housing 9 of the UAT unit in particular exemplary embodiments may also be coated with titanium dioxide, so that assemblies and parts of the system will be protected from exposure to UV radiation. In addition, if the UAT unit 8 is mounted directly in the mixing chamber 3, the protective grille 15 with the titanium dioxide coating can be installed only on the inlet window 10 for the recirculated air into the UAT unit, and the second grille on the air outlet window 11 may be omitted. In this case, the coating of titanium dioxide can also be applied to the internal surface of the air duct 9 of the UAT unit 8.

Also, as a design variant, it is possible to mount the protective grille 15 with the titanium dioxide coating only on the air outlet window 11 and not to mount the grille on the window 10. If necessary, it is also possible to coat the internal surface of the air-duct housing 9 with titanium dioxide. In some cases it is acceptable for the grilles 15 to be entirely omitted, wherein it must be ensured that a titanium dioxide coating has been applied to the internal surface of the air-duct housing 9 of the UAT unit 8. If the assemblies and parts of the ACS are made from materials that are resistant to exposure to UV radiation, and the UAT unit is mounted in the mixing chamber: protective grilles may be omitted, wherein a coating that reflects UV radiation is applied to the internal surface of the UAT unit, which allows the air treatment also to be performed outside the confines of the UAT unit 8.

It is also possible to install the protective grille with the titanium dioxide coating only on the inlet window 10 for the air to be treated into the UAT unit 8, and to apply a coating that reflects UV radiation to the internal surface of the UAT unit. In this case, the propagation of UV radiation in the direction of the mixing chamber 3 will be precluded if the materials used in its construction are not resistant to UV radiation, wherein the UV radiation will reach the main air duct 4 and partially reach as far as the evaporation unit 5, preventing the development of biological films consisting of micro-organisms on them.

If the UAT unit 8 is installed downstream of the mixing chamber 3 and upstream of the evaporation unit 5 and the materials used in the construction of the ACS are not resistant to exposure by UV radiation, then the protective grilles 15 coated with titanium dioxide are advantageously installed on both windows 10 and 11 of the UAT unit 8, wherein in some cases the titanium dioxide coating may also be applied to the internal surface of the air-duct housing 9 of the UAT unit 8, which will additionally protect assemblies and components of the system from exposure to UV radiation.

It is possible to install the protective grille with the titanium dioxide coating only on the inlet window 10 for the air to be treated into the UAT unit, and not to install the second grille on the air outlet window 11. In this case, to provide additional protection from UV radiation, a coating of titanium dioxide can be applied to the internal surface of the air-duct housing 9 of the UAT unit 8.

It is also possible to mount the protective grille 15 with the titanium dioxide coating only on the air outlet window 11. If so, in particular cases a titanium dioxide coating can be applied to the internal surface of the air-duct housing 9 of the UAT unit 8. In addition, the protective grilles can be entirely omitted, in which case the titanium dioxide coating is advantageously applied to the internal surface of the air-duct housing 9 of the UAT unit 8.

When the grilles are omitted, a design variant is also possible in which a coating which reflects UV radiation is applied to the internal surface of the UAT. In this case, the UV radiation that falls outside the UAT unit 8 will be effectively treated by the components of the evaporation unit 5 and the air exiting from the mixing chamber 3.

If the protective grille 15 with the titanium dioxide coating is installed only on the inlet window for the air to be treated into the UAT unit 8, and if a coating that reflects the UV radiation is applied to the internal surface of the UAT unit, then effective treatment will be applied not only to the air in the unit 8 but also to the components of the evaporation unit 5. If the protective grille with the titanium dioxide coating is installed only on the inlet window 11 for the air to be treated into the UAT unit 8, and a coating that reflects UV radiation is applied to the internal surface of the UAT unit, then effective treatment will be applied to the air present outside the confines of the unit 8 exiting from the mixing chamber 3.

If the UAT unit is mounted downstream of the evaporation unit and upstream of the inlet fan, the titanium dioxide-coated protective grilles can be installed on both windows of the UAT unit, i.e. on the air inlet window 10 into the UAT unit and on the air outlet window 11 in the direction of the inlet fan 6. To increase the absorption effect of the UV radiation and provide protection for the ACS components, a titanium dioxide coating can be applied to the internal surface of the air-duct housing 9 of the UAT unit 8.

As a design variant, the protective grille with titanium dioxide coating may be installed only on the inlet window 10 for the air to be treated into the UAT unit 8. In this connection, the titanium dioxide coating can in many cases also be applied to the internal surface of the air-duct housing 9 of the UAT unit 8.

If the protective grille with a coating of titanium dioxide is installed only on the air outlet window and a titanium dioxide coating is applied to the internal surface of the air-duct housing of the UAT unit, the inlet fan 6 will be more effectively protected.

In the absence of protective grilles, protection from UV radiation can be achieved by means of titanium dioxide coatings applied to the internal surface of the air-duct housing 9 of the UAT unit 8. If the protective grilles are omitted and a coating that reflects the UV radiation is applied to the internal surface of the air-duct housing 9 of the UAT unit, then in addition to the air in the UAT unit the surfaces of the inlet fan 6 and the evaporation unit 5 will also be effectively treated, which is possible if no materials are used in their construction that are destroyed under exposure to UV radiation.

If the protective grille 15 with the titanium dioxide coating is installed only on the inlet window 10 for the air to be treated into the UAT unit 8, and a coating that reflects UV radiation is applied to the internal surface of the UAT unit, then in addition to the air being treated in the UAT unit 8, UV radiation will also reach the inlet fan 6, thereby treating its surface.

If the UAT unit is mounted downstream of the inlet fan, the protective grilles with the titanium dioxide coating can be installed on the air inlet window 10 into the UAT unit and on the air outlet window 11 in the direction of the inlet fan 6. Additionally, a titanium dioxide coating could be applied to the internal surface of the air-duct housing 9 of the UAT unit 8, thereby precluding the release of UV radiation beyond the confines of the UAT unit. If the protective grille 15 with a titanium dioxide coating is only installed on the inlet window 10 for the air to be treated into the UAT unit, a titanium dioxide coating can be advantageously applied to the internal surface of the air-duct housing 9 of the UAT unit, as this will ensure a reduction of the UV radiation emitted in the direction of the passenger compartment.

The device works as follows. With the inlet fan 6 switched on, air is pumped along the entire length of the air conditioning system, namely from the air intake units for the external and recirculated air, both flows reach the mixing chamber 3, then pass along the main air duct 4 through the evaporation unit 5 and the inlet fan 6 into the forced-draft air duct 7, from where the air is supplied to the railway passenger compartment through the outlet window.

The UAT unit 8 can be installed in any position in the system - in the air intake unit for the recirculated air up to the mixing chamber 3, directly in the mixing chamber 3, or downstream of this in the main air duct 4, upstream of the evaporation unit 5, as well as downstream of the evaporation unit 5 and upstream of the inlet fan 6. The UAT unit may also be installed downstream of the inlet fan 6 in the forced-draft air duct 7. In addition, it is possible to use two, three or more UAT units installed in the ACS in any combination of the locations specified above, or simultaneously in all these locations.

In the UAT unit the air flow is irradiated by the ultraviolet amalgam lamp 13, or lamps if there are more than one, and the physical construction of the unit with protective grilles 15, coated with titanium dioxide and mounted on the inlet 10 and/or outlet windows 11 of the unit 8 in combination with the same coating on the inside of the air-duct housing 9 of the unit 8 (which is applied in particular embodiments of the unit), safely contain the UV radiation within the UAT unit 8. As a result, the main assemblies and components of the ACS are protected from UV radiation while at the same time preventing the release of radiation outside of the ACS and thereby any effect on the people present in the railway car compartment. With a coating reflecting UV radiation applied to the internal surface of the unit, the conditions are created for the destruction of bacteria, fungi, protozoa and viruses, because the UV radiation not only acts within the confines of the UAT unit 8 but is also emitted outside of them, treating the surrounding surfaces, which increases the likelihood of the destruction of the living micro-organisms in the air during their passage through the ACS system. More detailed design variants for the application of the coatings and the effects of such application are set out above in the description of the ACS.

### Exemplary embodiments of the invention.

### Example 1.

The ACS is an air conditioning system (device) equipped with an ultraviolet air treatment unit 8 (UAT unit), mounted upstream of a chamber 3. The UAT unit is connected to adjacent parts (air duct 1 and chamber 3) of the device, preferably by a flange connection, and is installed in accordance with the general configuration of an ACS. The UAT, in particular, can also be used as the inlet air duct for recirculated air, while at the same time ensuring a supply of recirculated air to the mixing chamber and the disinfection of the recirculated air. In this case the UAT unit is rigidly connected to the mixing chamber and to the recirculated air duct.

On the inlet and outlet windows 10 and 11 of the air-duct housing 9 of the unit 8, means for protection against UV radiation are mounted in the form of protective profiled grilles 15. The protective grilles 15 are installed on both windows of the UAT unit 8, i.e. on the inlet window 10 for the recirculated air and on the outlet window 11 for air into the mixing chamber 3, and their surface is provided with a photocatalytic coating based on titanium dioxide (TiO₂), which absorbs UV radiation and additionally improves the effectiveness of the disinfection and cleaning of the air due to the photocatalysis.

In addition, in a particular embodiment, the titanium dioxide (TiO₂) coating can be applied on the internal surface of the air-duct housing 9 of the UAT unit 8, which means it is possible to provide significant reduction in the UV radiation emission in the direction of the passenger compartment of the railway car because of the dual protection: UV radiation is absorbed by the coating of the internal surface of the air-duct housing 9 and of the protective grille 15 on the air inlet window 10. This prevents the emission of UV radiation outside the confines of the ACS through the inlet window for the recirculated air, so that the UV radiation does not reach the passengers present in the compartment. The protective grille 15 on the air outlet window 11 prevents the release of UV radiation in the direction of the mixing chamber 3, protecting the assemblies and parts of this section of the ACS from the damaging effects of UV radiation on some of the parts of the chamber 3, in so far as their construction can include materials which are unstable under the effects of UV radiation, such as galvanized steel, and porous rubber, which is used as a sealant material, etc.

With the inlet fan 6 switched on, air is pumped along the entire length of the air duct of the ACS, and namely both streams of the external and recirculated air from the air intake units 2 and 1 reach the mixing chamber 3. However, the air flow from the inlet recirculated air duct to the mixing chamber 3 reaches the UAT unit 8, where it is treated by the UV radiation from the lamp 13; then the decontaminated air, once it has reached the unit 8 from the inlet recirculation air duct, passes into the mixing chamber 3 where it is mixed with the external air. Then, the airflow along the main air duct 4 through the evaporation unit 5 and the inlet fan 6 enters the forced-draft air duct 7, from where cleaned and decontaminated air is fed into the passenger railway car through the outlet window.

In the UAT unit the air flow is irradiated by the ultraviolet amalgam lamp 13, or lamps if there are more than one, and the physical construction of the unit with protective grilles 15, coated with titanium dioxide and mounted on the inlet 10 and outlet windows 11 of the unit 8 in combination with the same coating on the inside of the air-duct housing 9 of the unit 8 (which is applied in particular embodiments of the unit 8), safely contain the UV radiation within the UAT unit 8. As a result, the main assemblies and components of the ACS are protected from UV radiation, while at the same time preventing the release of radiation outside of the unit 8 and thereby any effect on the people present in the railway car compartment.

### Example 2.

The ACS is an air conditioning system (device) equipped with an ultraviolet air treatment unit 8 (UAT unit), mounted upstream of a chamber 3. The UAT unit is connected to adjacent parts (air duct 1 and chamber 3) of the ACS, preferably by a flange connection, and is installed in accordance with the general configuration of an ACS. The UAT unit 8, in particular, can also be used as the inlet air duct for recirculated air, while at the same time ensuring the supply of the recirculated air to the mixing chamber and its disinfection.

On the inlet window 10 of the air-duct housing 9 of the unit 8, means for protection against UV radiation are mounted, in the form of a protective profiled grille 15. The protective grille 15 is mounted and secured on the air inlet window 10 of the UAT unit 8, and its surface is provided with a photocatalytic coating based on titanium dioxide (TiO₂), which absorbs UV radiation and additionally improves the effectiveness of the disinfection and cleaning of the air due to the photocatalysis.

In addition, in a particular embodiment, the titanium dioxide (TiO₂) coating can be applied on the internal surface of the air-duct housing 9 of the UAT unit 8, which means it is possible to provide significant reduction in the UV radiation emission in the direction of the passenger compartment of the railway car due to the dual protection - the UV radiation is absorbed by the coating of the internal surface of the air-duct housing 9 and of the protective grille 15 on the air inlet window 10. This is to prevent the emission of UV radiation outside the confines of the ACS through the inlet window for the recirculated air, so that UV radiation does not reach the passengers present in the compartment. If a protective titanium dioxide (TiO₂) coating is applied to the internal surface of the air-duct housing of the unit 8, this also practically prevents the release of UV radiation in the direction of the mixing chamber 3, protecting the assemblies and parts of this section of the ACS from the damaging effects of UV radiation, which is necessary due to the fact that materials may be used in the construction of the device which are unstable under the effects of UV radiation, such as galvanized steel, and porous rubber which is used as a sealant material, etc. If the mixing chamber is implemented from materials that are resistant to UV radiation, no TiO₂-based coating needs to be applied to the internal surface of the air-duct housing of the unit 8. In this case, the UV radiation will extend beyond the unit 8, and the treatment will be applied to the surfaces located outside the unit 8, wherein the air treatment zone also extends beyond the confines of the unit 8, which will have a positive impact on the disinfection results.

With the inlet fan 6 switched on, air is pumped along the entire length of the air duct of the air conditioning system, and both streams of the external and recirculated air reach the mixing chamber 3 from the air intake units 2 and 1. However, the air flow from the inlet recirculated air duct to the mixing chamber 3 reaches the UAT unit 8, where it is treated by the UV radiation from the lamp 13, and only then does the decontaminated air, once it has reached the unit 8 from the inlet recirculation air duct 1, pass into the mixing chamber 3 where it is mixed with the external air. Then, the airflow along the main air duct 4 through the evaporation unit 5 and the inlet fan 6 enters the forced-draft air duct 7, from where cleaned and decontaminated air is fed into the passenger railway car through the outlet window. In the UAT unit, the air flow is irradiated by the ultraviolet amalgam lamp 13, or lamps if there are more than one, and the physical construction of the unit with the protective grille 15 coated with titanium dioxide and mounted on the inlet window 10 of the unit 8, in combination with the same coating on the inside of the air-duct housing 9 of the UAT unit 8 (which is applied in particular embodiments of the unit 8), safely contain the UV radiation within the UAT unit 8. As a result of the UV radiation, both the major components and parts of the device are protected, and release of radiation outside of the ACS in the direction of the passenger compartment is prevented, which precludes any impact on the people present in the compartment of the railway car.

### Example 3.

The ACS is an air conditioning system (device) equipped with an ultraviolet air treatment unit 8 (UAT unit), mounted in the mixing chamber 3, in which the unit 8 has a flange 14 for securing the unit to the mixing chamber and is rigidly connected thereto.

On the inlet and outlet windows 10 and 11 of the air-duct housing 9 of the unit 8, located in the chamber 3, means for protection against UV radiation in the form of protective profiled grilles 15 are installed. The protective grilles 15 are installed on both windows of the UAT unit 8, i.e. on the air inlet window 10 and on the air outlet window 11, and their surface is provided with a photocatalytic coating based on titanium dioxide (TiO₂), which absorbs UV radiation and additionally improves the effectiveness of the disinfection and cleaning of the air due to the photocatalysis.

In addition, in a particular embodiment, the titanium dioxide (TiO₂) coating can be applied to the internal surface of the air-duct housing 9 of the UAT unit 8, as a result of which a significant reduction of the UV radiation emission beyond the confines of the UAT unit 8 can be achieved.

With the inlet fan 6 switched on, air is pumped along the entire length of the air duct of the air conditioning system, and both streams of the external and recirculated air reach the mixing chamber 3 from the air intake units 2 and 1. In the UAT unit installed in the mixing chamber 3, the air flow is irradiated by the ultraviolet amalgam lamp 13, or lamps if there are more than one, and the physical construction of the unit with protective grilles 15 coated with titanium dioxide and mounted on the inlet 10 and outlet windows 11 of the unit 8, in combination with the same coating on the inside of the air-duct housing 9 of the unit 8 (which is applied in particular embodiments of the unit 8), safely contain the UV radiation within the UAT unit 8. As a result, the main assemblies and components of the mixing chamber are protected from UV radiation while the release of radiation outside of the UAT unit is prevented. Further along the main air duct 4 the airflow passes through the evaporation unit 5 and the inlet fan 6 to enter the forced-draft air duct 7, from where the cleaned and decontaminated air is fed into the passenger railway car through the outlet window of the device.

### Example 4.

The ACS is an air conditioning system (device) equipped with an ultraviolet air treatment unit 8 (UAT unit), mounted in the mixing chamber 3, in which the unit 8 has a flange 14 for securing the unit to the mixing chamber and is rigidly connected thereto.

On the air inlet window 10 of the air-duct housing 9 of the unit 8, a means for protecting against UV radiation is mounted, in the form of a protective profiled grille 15. The protective grille 15 is mounted on the air inlet window 10 of the UAT unit 8, and its surface is provided with a photocatalytic coating based on titanium dioxide (TiO₂), which absorbs UV radiation and additionally improves the effectiveness of the disinfection and cleaning of the air due to the photocatalysis. On the air outlet window 11 in the UAT unit 8, the protective grille is omitted.

In addition, in a particular embodiment, the titanium dioxide (TiO₂) coating can be applied on the internal surface of the air-duct housing 9 of the UAT unit 8, which means it is possible to provide significant reduction in the UV radiation emission in the direction of the passenger compartment of the railway car due to the dual protection: the UV radiation is absorbed by the coating of the internal surface of the air-duct housing 9 and of the protective grille 15 on the air inlet window 10, which prevents the emission of UV radiation outside the confines of the ACS through the inlet window for the recirculated air, so that the UV radiation does not reach the passengers present in the compartment. If a protective TiO₂ coating is applied to the internal surface of the air-duct housing of the unit 8, this likewise prevents the release of UV radiation in the direction of the mixing chamber 3, protecting the assemblies and components of the device from the damaging effects of UV radiation, in so far as materials may be used in the construction of the device which are unstable under the effects of UV radiation, such as galvanized steel, and porous rubber which is used as a sealant material, etc. If the mixing chamber 3 is implemented from materials that are resistant to UV radiation, no TiO₂-based coating needs to be applied to the internal surface of the air-duct housing of the unit 8. In this case, the UV radiation will extend beyond the unit 8, and the treatment will be applied to the surfaces located outside the unit 8, but the air treatment zone also extends beyond the confines of the unit 8, which will have a positive impact on the disinfection results.

The device works as follows. With the inlet fan 6 switched on, air is pumped along the entire length of the air duct of the air conditioning system, and both streams of the external and recirculated air from the air intake units 2 and 1 reach the mixing chamber 3. In the UAT unit installed in the mixing chamber 3, the air flow is irradiated by the ultraviolet amalgam lamp 13, or lamps if there are more than one, and the physical construction of the unit with protective grilles 15 coated with titanium dioxide and mounted on the inlet window 10 of the unit 8, in combination with the same coating on the inside of the air-duct housing 9 of the unit 8 (which is applied in particular embodiments of the unit 8), safely contain the UV radiation within the UAT unit. Due to the installation of the protective grille on the air inlet window, the sensitive assemblies and components of the system are protected from the UV radiation, while radiation emission outside of the device and any impact it may have on the people present in the cabin railway car are prevented. If there is no coating on the inside of the air-duct housing 9 of the unit 8, the UV radiation will be released outside the confines of the unit 8 in the direction of the air stream and will treat both the surfaces located outside of the unit 8 and continue to treat the air emitted from the unit 8. Further along the main air duct 4 from the mixing chamber 3, the airflow passes through the evaporation unit 5 and the inlet fan 6 to enter the forced-draft air duct 7, from where the cleaned and decontaminated air is fed into the passenger railway car through the outlet window of the device.

### Example 5.

The ACS is an air conditioning system (device) equipped with an ultraviolet air treatment unit 8 (UAT unit), mounted in the mixing chamber 3, in which the unit 8 has a flange 14 for securing the unit to the mixing chamber and is rigidly connected thereto.

On the outlet window 11 of the air-duct housing 9 of the unit 8, a means for protecting against UV radiation is mounted, in the form of a protective profiled grille 15. The protective grille 15 is mounted on the air outlet window 11 of the UAT unit 8, and its surface is provided with a photocatalytic coating based on titanium dioxide (TiO₂), which absorbs UV radiation and additionally improves the effectiveness of the disinfection and cleaning of the air due to the photocatalysis.

In addition, in a particular embodiment, the titanium dioxide (TiO₂) coating can be applied to the internal surface of the air-duct housing 9 of the UAT unit 8, which enables a significant reduction of the UV radiation emission in the direction of the passenger compartment through the recirculated air outlet window to be achieved. If a protective TiO₂ coating is applied to the internal surface of the air-duct housing of the unit 8, this likewise significantly reduces the emission of UV radiation in the direction of the mixing chamber 3, protecting the assemblies and components of the device from the damaging effects of UV radiation, in so far as materials may be used in the construction of the device which are unstable under the effects of UV radiation, such as galvanized steel, and porous rubber which is used as a sealant material, etc.

The device works as follows. With the inlet fan 6 switched on, air is pumped along the entire length of the air duct of the air conditioning system, and both streams of the external and recirculated air from the air intake units 2 and 1 reach the mixing chamber 3. In the UAT unit installed in the mixing chamber 3, the air flow is irradiated by the ultraviolet amalgam lamp 13, or lamps if there are more than one, and the physical construction of the unit with a protective grille 15 coated with titanium dioxide and mounted on the outlet window 11 of the unit 8, in combination with the same coating on the inside of the air-duct housing 9 of the unit 8 (which is applied in particular embodiments of the unit 8), safely contain the UV radiation within the UAT unit 8. If there is no coating on the inside of the air-duct housing 9 of the unit 8, the UV radiation will be released outside the confines of the unit 8 in the opposite direction to the air flow and will treat both the surfaces located upstream of the unit 8 and also the air emitted into the unit 8. As a result of the presence of the protective grille on the air outlet window, the sensitive assemblies and parts of the system are protected from UV radiation from the direction of the mixing chamber 3, and the air treatment zone extends beyond the UAT unit against the direction of air flow. Further along the main air duct 4 from the mixing chamber 3, the airflow passes through the evaporation unit 5 and the inlet fan 6 to enter the forced-draft air duct 7, from where the cleaned and decontaminated air is fed into the passenger railway car through the outlet window of the device.

### Example 6.

The ACS is an air conditioning system (device) equipped with an ultraviolet air treatment unit 8 (UAT unit), mounted in the mixing chamber 3, in which the unit 8 has a flange 14 for securing the unit to the mixing chamber and is rigidly connected thereto. The UAT unit 8 consists of an air-duct housing 9 having windows 10 and 11 for the inlet and outlet respectively of the air to be treated. The protective grilles on the windows 10 and 11 are omitted. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit 8 has flanges 14 for securing the unit to the mixing chamber and is rigidly connected to the mixing chamber 3, forming an integral unit with it.

In addition, in a particular embodiment, the titanium dioxide (TiO₂) coating can be applied to the internal surface of the air-duct housing 9 of the UAT unit 8, as a result of which a significant reduction of the UV radiation emission beyond the confines of the UAT unit 8 can be achieved in addition to improved effectiveness of the disinfection and cleaning of the air due to the photocatalysis.

The titanium dioxide-based coating prevents the emission of UV radiation outside the confines of the device through the air inlet window, so that UV radiation does not reach the passengers present in the compartment. In addition, if a coating based on TiO₂ is applied to the internal surface of the air-duct housing of the unit 8, this will prevent the emission of UV radiation in the direction of the mixing chamber 3, protecting the assemblies and components of the device from the destructive effects of the UV radiation. This is necessary because materials which are not resistant to the action of UV radiation may also be used in the construction of the device, such as galvanized steel, and porous rubber, which is used as a sealing material for the maintenance hatch, etc. If the mixing chamber 3 is implemented from materials that are resistant to UV radiation, no TiO₂-based coating needs to be applied to the internal surface of the air-duct housing of the unit 8. In this case, the UV radiation will extend beyond the unit 8, and the treatment will be applied to the surfaces located both upstream of the unit 8 and downstream of the unit 8, but the air treatment zone will also extend beyond the confines of the unit 8, which will have a positive impact on the disinfection results.

The device works as follows. With the inlet fan 6 switched on, air is pumped along the entire length of the air duct of the air conditioning system, and both streams of the external and recirculated air from the air intake units 2 and 1 reach the mixing chamber 3. In the UAT unit installed in the mixing chamber 3, the air flow is irradiated by the ultraviolet amalgam lamp 13, or lamps if there are more than one, and the physical construction of the unit with a coating of TiO₂ on the inside of the air-duct housing 9 of the unit 8 (which is applied in particular embodiments of the unit 8), safely confines the UV radiation within the UAT unit 8. As a result, the sensitive assemblies and components of the system are protected against UV radiation from the direction of the inlet recirculated air duct 1, which also prevents radiation emission outside the confines of the device and any impact it may have on people present in the cabin railway car. If there is no coating on the inside of the air-duct housing 9 of the unit 8, the UV radiation will be released outside the confines of the unit 8 along the direction of the air flow and in the opposite direction, which allows the surfaces located outside of the unit 8 to be treated, and the distance over which the air is treated to be increased, i.e. not only within the unit 8, but also before entering and after exiting the unit 8. Further along the main air duct 4 from the mixing chamber 3, the airflow passes through the evaporation unit 5 and the inlet fan 6 to enter the forced-draft air duct 7, from where the cleaned and decontaminated air is fed into the passenger railway car through the outlet window of the device.

### Example 7.

The ACS is an air conditioning system (device) equipped with an ultraviolet air treatment unit 8 (UAT unit), mounted in the mixing chamber 3, in which the unit 8 has a flange 14 for securing the unit to the mixing chamber and is rigidly connected thereto. The UAT unit 8 consists of an air-duct housing 9 having windows 10 and 11 for the inlet and outlet respectively of the air to be treated. The protective grilles on the windows 10 and 11 are omitted. Lamp assemblies 12 with electrical connectors for one, two or more sources of germicidal UV radiation - in the form of amalgam lamps 13, primarily having a U-shaped form - are secured to the wall of the air-duct housing of the UAT unit.

The coating reflecting the UV radiation which is applied to the internal surface of the air-duct housing 9 of the UAT unit 8, which additionally enables the efficiency of the disinfection and cleaning of the air to be increased, due to the fact that the UV radiation will extend beyond the unit 8, and the treatment will be applied to surfaces located in front of the unit 8 and behind the unit 8, including the air treatment zone also extending beyond the confines of the unit 8, which has a positive impact on the disinfection results.

In the construction of the air conditioning device, in areas adjacent to the mixing chamber 3, materials resistant to UV radiation are used.

The device works as follows. With the inlet fan 6 switched on, air is pumped along the entire length of the air duct of the air conditioning system, and both streams of the external and recirculated air from the air intake units 2 and 1 reach the mixing chamber 3. In the UAT unit installed in the mixing chamber 3, the air flow is irradiated by an ultraviolet amalgam lamp 13, or lamps if there are more than one, and the embodiment of the unit with a coating reflecting UV radiation on the inside of the air-duct housing 9 of the unit 8 allows the radiation to be emitted beyond the unit 8 in the direction of the air flow and in the opposite direction, which causes treatment of the surfaces located outside the unit 8, as well as the air supplied into the unit 8 and exiting from it. Further along the main air duct 4 from the mixing chamber 3, the airflow passes through the evaporation unit 5 and the inlet fan 6 to enter the forced-draft air duct 7, from where the cleaned and decontaminated air is fed into the passenger railway car through the outlet window of the device.

### Example 8.

The ACS is an air conditioning system (device) equipped with an ultraviolet air treatment unit 8 (UAT unit), mounted in the mixing chamber 3, in which the unit 8 has a flange 14 for securing the unit to the mixing chamber and is rigidly connected thereto. The UAT unit 8 consists of an air-duct housing 9 having windows 10 and 11 for the inlet and outlet respectively of the air to be treated. Lamp assemblies 12 with electrical connectors for one, two or more sources of germicidal UV radiation - in the form of amalgam lamps 13, primarily having a U-shaped form - are secured to the wall of the air-duct housing of the UAT unit. On the inlet window 10 of the air-duct housing 9 of the unit 8, means for protection against UV radiation are mounted, in the form of a protective profiled grille 15. The protective grille 15 is mounted on the air inlet window 10 of the UAT unit 8, and its surface is provided with a photocatalytic coating based on titanium dioxide (TiO₂), which absorbs UV radiation and additionally improves the effectiveness of the disinfection and cleaning of the air due to the photocatalysis.

As a result, the sensitive assemblies and components of the system located upstream of the mixing chamber 3 are protected against UV radiation, which prevents radiation emission outside the confines of the device and any impact it may have on people present in the cabin railway car.

In addition, a coating reflecting UV radiation is applied to the inside surface of the air-duct housing 9 of the UAT unit 8. Thus, on the one hand, the emission of UV radiation outside the confines of the ACS through the air inlet window into the unit 8 is prevented (due to the protective grille with the TiO₂ coating) and reflected UV radiation is prevented from reaching the passengers present in the compartment, and on the other hand, the coating reflecting the UV radiation, which is applied to the internal surface of the air-duct housing 9 of the UAT unit 8, creates the conditions for the propagation of the UV radiation beyond the confines of the unit 8, and the treatment will be applied to surfaces located behind the unit 8 and the air treatment zone will also extend beyond the confines of the unit 8, which has a positive impact on the disinfection results.

The device works as follows. With the inlet fan 6 switched on, air is pumped along the entire length of the air duct of the air conditioning system, and both streams of the external and recirculated air from the air intake units 2 and 1 reach the mixing chamber 3. In the UAT unit 8 installed in the mixing chamber 3, the air flow is irradiated by the ultraviolet amalgam lamp 13, or lamps if there are more than one, and the physical construction of the unit with the protective grille 15 coated with titanium dioxide and mounted on the inlet window 10 of the unit 8, in combination with the coating that reflects UV radiation applied to the inside surface of the air-duct housing 9 of the unit 8, does not allow radiation to be emitted in the direction of the recirculated air inlet 1, and at the same time allows the treatment zone for the air and the surfaces located outside the UAT unit 8 to be extended in the direction of the air flow, increasing the likelihood of destroying the living micro-organisms in the air stream. Further along the main air duct 4 from the mixing chamber 3, the airflow passes through the evaporation unit 5 and the inlet fan 6 to enter the forced-draft air duct 7, from where the cleaned and decontaminated air is fed into the passenger railway car through the outlet window of the device.

### Example 9.

The ACS is an air conditioning system (device) equipped with an ultraviolet air treatment unit 8 (UAT unit), mounted downstream of a mixing chamber 3 and upstream of an evaporation unit 5. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected, for example, to adjacent parts of the main air duct 4 of the device, preferably by a flange connection, and is mounted between the mixing chamber and the main air duct in accordance with the general configuration of an ACS.

On the inlet and outlet windows 10 and 11 of the air-duct housing 9 of the unit 8, means for protection against UV radiation are mounted in the form of protective profiled grilles 15. Protective grilles 15 are installed on both windows of the UAT unit 8, i.e. on the air inlet window 10 from the mixing chamber 3 and on the air outlet window 11 for the treated air in the direction of the evaporation unit 5. The surfaces of the protective grilles 15 are each provided with a photocatalytic coating based on titanium dioxide (TiO₂) that absorbs UV radiation, and additionally improves the effectiveness of the disinfection and cleaning of the air due to photocatalysis.

In addition, in a particular embodiment, the titanium dioxide (TiO₂) coating can be applied on the internal surface of the air-duct housing 9 of the UAT unit 8, which means it is possible to provide significant reduction in the UV radiation emission in both directions from the unit 8 due to the dual protection: the UV radiation is absorbed by the coating of the internal surface of the air-duct housing 9 and by the protective grilles 15 on the air inlet and outlet windows 10 and 11. This prevents the emission of UV radiation beyond the confines of the UAT unit 8, which protects the assemblies and components of the device from the damaging effects of the UV radiation. This is necessary because materials which are not resistant to the action of UV radiation may be used in the construction of the device, such as galvanized steel, porous rubber, which is used as a sealing material for maintenance hatches etc.

The device works as follows. With the inlet fan 6 switched on, air is pumped along the entire length of the air duct of the air conditioning system, and both streams of the external and recirculated air from the air intake units 2 and 1 reach the mixing chamber 3. Then, from the mixing chamber 3 the air flow passes into the UAT unit 8 downstream of the mixing chamber 3. In the UAT unit 8 the air is continuously treated by the UV radiation from the lamp 13; the decontaminated air, having entered the unit 8 from the mixing chamber 3, then reaches the evaporation unit 5 and via the evaporation unit 5 and the inlet fan 6, enters the forced-draft air duct 7, from where through the exit window the cleaned and decontaminated air is fed into the passenger railway car.

In the UAT unit 8 the air flow is irradiated by the ultraviolet amalgam lamp 13, or lamps if there are more than one, and the physical construction of the unit with the two protective grilles 15 coated with titanium dioxide and mounted on the inlet 10 and outlet windows 11 of the unit 8, in combination with the same coating on the inside of the air-duct housing 9 of the unit 8 (which is applied in particular embodiments of the unit 8), safely contain the UV radiation within the UAT unit. As a result, the main assemblies and components of the device are protected from UV radiation, while at the same time preventing the release of radiation outside of the unit 8 and thereby any effect on the people present in the railway car compartment.

### Example 10.

The ACS is an air conditioning system (device) equipped with an ultraviolet air treatment unit 8 (UAT unit), mounted downstream of a mixing chamber 3 and upstream of an evaporation unit 5. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to adjacent parts of the main air duct 4 of the ACS, preferably by a flange connection, and is mounted between the mixing chamber and the main air duct in accordance with the general configuration of an ACS.

On the inlet window 10 of the air-duct housing 9 of the unit 8, means for protection against UV radiation are mounted, in the form of a protective profiled grille 15. The protective grille 15 is mounted on the recirculated air inlet window 10 of the UAT unit 8, and its surface is provided with a photocatalytic coating based on titanium dioxide (TiO₂), which absorbs UV radiation and additionally improves the effectiveness of the disinfection and cleaning of the air due to the photocatalysis.

In addition, in a particular embodiment, the titanium dioxide (TiO₂) coating can be applied on the internal surface of the air-duct housing 9 of the UAT unit 8, which means it is possible to provide a significant reduction in the UV radiation emission in the direction of the mixing chamber 3 due to the dual protection - the UV radiation is absorbed by the coating on the internal surface of the air-duct housing 9 and on the protective grille 15 on the air inlet window 10. This prevents the emission of UV radiation beyond the confines of the device through the air inlet window and eliminates the negative effects of the UV radiation on the parts and materials that are not resistant to the ultraviolet light. If a coating based on TiO₂ is additionally applied to the internal surface of the air-duct housing of the unit 8, this will even more effectively prevent the emission of UV radiation in the direction of the mixing chamber 3, protecting the assemblies and components of the device from the destructive effects of the UV radiation. The emission of UV radiation in the direction of the evaporation unit 5 is also limited. This can be important if materials which are not resistant to the action of UV radiation were used in the construction of the ACS, such as galvanized steel, porous rubber, which is used as a sealing material for the maintenance hatches etc.

If the evaporation unit is implemented from materials that are resistant to UV radiation, then no TiO₂-based coating needs to be applied to the internal surface of the air-duct housing of the unit 8. In this case, the UV radiation in the direction of the mixing chamber will be blocked and will extend beyond the unit 8 in the direction of the evaporation unit 5 along the direction of motion of the air stream, and the treatment will be applied to the surface of the evaporation unit 5 located downstream of the unit 8, wherein the air treatment zone will also extend beyond the confines of the unit 8, and the time over which it is treated with UV will increase, which has a positive impact on the disinfection results.

The device works as follows. With the inlet fan 6 switched on, air is pumped along the entire length of the air duct of the air conditioning system, and both streams of the external and recirculated air from the air intake units 2 and 1 reach the mixing chamber 3. The UAT unit 8 is installed downstream of the mixing chamber 3 and upstream of the evaporation unit. In the UAT unit, the air flow is irradiated by the ultraviolet amalgam lamp 13, or lamps if there are more than one, and the physical construction of the unit with the protective grille 15 coated with titanium dioxide and mounted on the inlet window 10 of the unit 8, in combination with the same coating on the inside of the air-duct housing 9 of the unit 8 (which is applied in particular embodiments of the unit 8), safely contain the UV radiation within the UAT unit 8. As a result, the major components and assemblies of the air conditioning system are protected from UV radiation. Then, the airflow along the main air duct 4 through the evaporation unit 5 and the inlet fan 6 enters the forced-draft air duct 7, from where cleaned and decontaminated air is fed into the passenger railway car through the outlet window.

### Example 11.

The ACS is an air conditioning system (device) equipped with an ultraviolet air treatment unit 8 (UAT unit), mounted downstream of a mixing chamber 3 and upstream of an evaporation unit 5. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to adjacent parts of the main air duct 4 of the device, preferably by a flange connection, and is mounted between the mixing chamber and the main air duct in accordance with the general configuration of an ACS.

On the outlet window 11 of the air-duct housing 9 of the unit 8, a means for protecting against UV radiation is installed, in the form of a protective profiled grille 15. The protective grille 15 is mounted on the air outlet window 11, and its surface is provided with a photocatalytic coating based on titanium dioxide (TiO₂), which absorbs UV radiation and additionally improves the effectiveness of the disinfection and cleaning of the air due to the photocatalysis.

In addition, in a particular embodiment, the titanium dioxide (TiO₂) coating can be applied to the internal surface of the air-duct housing 9 of the UAT unit 8, which means it is possible to provide a significant reduction in the UV radiation emission in the direction of the evaporation unit 5 due to the dual protection: the UV radiation is absorbed by the coating on the internal surface of the air-duct housing 9 and on the protective grille 15 on the air outlet window 11. If a protective TiO₂ coating is applied to the internal surface of the air-duct housing of the unit 8, this also prevents the emission of UV radiation in the direction of the mixing chamber 3, protecting its assemblies and parts from exposure to the UV radiation; this can be necessary due to the fact that materials may be used in the construction of the chamber 3 which are unstable under the effects of UV radiation, such as galvanized steel and porous rubber, which is used as a sealant material, etc.

If the mixing chamber 3 is implemented from materials that are resistant to UV radiation, no TiO₂-based coating needs to be applied to the internal surface of the air-duct housing of the unit 8. In this case, the UV radiation will propagate beyond the confines of the unit 8, and the treatment will be applied to the surfaces located upstream of the unit 8, but the air treatment zone will also extend beyond the confines of the unit 8, in the opposite direction to the movement of the air stream, thus increasing the treatment time, which will have a positive impact on the disinfection results.

The device works as follows. With the inlet fan 6 switched on, air is pumped along the entire length of the air duct of the air conditioning system, and both streams of the external and recirculated air from the air intake units 2 and 1 reach the mixing chamber 3. In the UAT unit installed downstream of the mixing chamber and upstream of the evaporation unit and installed between the two, for example, on account of the flange connections, the air flow is irradiated by the ultraviolet amalgam lamp 13, or lamps if there are more than one, and the physical construction of the unit with a protective grille 15 coated with titanium dioxide and mounted on the outlet window 11 of the unit 8, in combination with the same coating on the inside of the air-duct housing 9 of the unit 8 (which is applied in particular embodiments of the unit 8), safely contain the UV radiation within the UAT unit 8. As a result, the sensitive components and assemblies of the system, in particular, the components of the evaporation unit 5 and the components of the mixing chamber 3, are protected from UV radiation. If there is no coating on the inside of the air-duct housing 9 of the unit 8, the UV radiation will be emitted outside the confines of the unit 8 in the opposite direction to the air flow and will treat the surfaces outside the unit 8 (the mixing chamber 3, the main air duct 4) and also the air entering the unit 8.

Then, the airflow through the evaporation unit 5 and the inlet fan 6 enters the forced-draft air duct 7, from where the cleaned and decontaminated air is fed into the passenger railway car through the outlet window.

### Example 12.

The ACS is an air conditioning system (device) equipped with an ultraviolet air treatment unit 8 (UAT unit), mounted downstream of a mixing chamber 3 and upstream of an evaporation unit 5. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to adjacent parts of the main air duct 4 of the device, preferably by a flange connection, and is mounted between the mixing chamber and the main air duct in accordance with the general configuration of an ACS. The protective grilles in the construction of the UAT unit 8 are omitted.

In a particular embodiment, the titanium dioxide (TiO₂) coating can be applied to the internal surface of the air-duct housing 9 of the UAT unit 8, which results in a significant reduction of the UV radiation emission beyond the confines of the UAT unit 8 being achieved, in addition to improved effectiveness of the disinfection and cleaning of the air due to the photocatalysis.

Due to the titanium dioxide-based coating, the emission of UV radiation beyond the UAT unit is reduced, since if a coating based on TiO₂ is applied on the internal surface of the air-duct housing of the unit 8, this prevents the emission of UV radiation in the direction of the mixing chamber 3 and the evaporation unit 5, protecting the assemblies and components of the air conditioning system from the damaging effects of UV radiation. This is necessary because materials which are not resistant to the action of UV radiation may also be used in the construction of the air conditioning system, such as galvanized steel, porous rubber, which is used as a sealing material etc. If the mixing chamber 3 and the evaporation unit 5 are implemented from materials that are resistant to UV radiation, no TiO₂-based coating needs to be applied to the internal surface of the air-duct housing of the unit 8. In this case, the UV radiation will propagate beyond the confines of the unit 8, and the treatment will be applied to the surfaces located both upstream of the unit 8 and downstream of the unit 8, but the range and duration of the air treatment will also extend beyond the confines of the UAT unit 8, which will have a positive impact on the disinfection results.

The device works as follows. With the inlet fan 6 switched on, air is pumped along the entire length of the air duct of the air conditioning system, and both streams of the external and recirculated air from the air intake units 2 and 1 reach the mixing chamber 3 and then to the UAT unit 8. In the UAT unit installed between the mixing chamber 3 and the evaporation unit 5, the air flow is irradiated by the ultraviolet amalgam lamp 13, or lamps if there are more than one, and the physical construction of the unit with a coating of TiO₂ on the inside of the air-duct housing 9 of the unit 8 (which is applied in particular embodiments of the unit 8), safely confines the UV radiation within the UAT unit 8. As a result, the sensitive assemblies and components of the air conditioning system are protected from the UV radiation. If there is no coating on the inside of the air-duct housing 9 of the unit 8, the UV radiation will be released outside the confines of the unit 8 along the direction of the air flow, towards the evaporation unit 5, and in the opposite direction, towards the mixing chamber 3, which allows the surfaces of the ACS located outside of the unit 8 to be treated, and also the distance and duration over which the air is treated to be increased, i.e. the treatment takes place not only in the unit 8, but also before entering and after exiting the unit 8. Further along the main air duct 4 from the UAT unit 8, the airflow passes through the evaporation unit 5 and the inlet fan 6 to enter the forced-draft air duct 7, from where the cleaned and decontaminated air is fed into the passenger railway car through the outlet window of the device.

### Example 13.

The ACS is an air conditioning system (device) equipped with an ultraviolet air treatment unit 8 (UAT unit), mounted downstream of a mixing chamber 3 and upstream of an evaporation unit 5. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to the adjacent parts of the main air duct 4 of the system, preferably by a flange connection, and is installed in accordance with the general configuration of an ACS. The protective grilles are omitted in the construction of the UAT unit 8.

The coating reflecting the UV radiation, which is applied to the internal surface of the air-duct housing 9 of the UAT unit 8, which additionally enables the efficiency of the disinfection and cleaning of the air to be increased, due to the fact that the UV radiation will propagate beyond the confines of the UAT unit 8, and the treatment will be extended to surfaces located both upstream of the unit 8 and downstream of the unit 8. The air treatment area is also extended beyond the confines of the unit 8, and the duration of the air treatment is increased, that will have a positive impact on the disinfection results.

In the construction of the air conditioning system, in areas adjacent to the UAT unit, materials resistant to UV radiation must be used.

The device works as follows. With the inlet fan 6 switched on, air is pumped along the entire length of the air duct of the air conditioning system, and both streams of the external and recirculated air from the air intake units 2 and 1 reach the mixing chamber 3, and then the air stream passes into the UAT unit 8. In the UAT unit installed between the mixing chamber 3 and the evaporation unit 5, the air flow is irradiated by an ultraviolet amalgam lamp 13, or lamps if there are more than one, and the embodiment of the unit with a coating reflecting UV radiation on the inside of the air-duct housing 9 of the unit 8 allows the radiation to be emitted beyond the confines of the unit 8 in the direction of the air flow and in the opposite direction, which causes treatment of the surfaces located outside of the unit 8, as well as the air supplied into the unit 8 and exiting from it. Further along the main air duct 4 from the UAT unit, the airflow passes through the evaporation unit 5 and the inlet fan 6 to enter the forced-draft air duct 7, from where the cleaned and decontaminated air is fed into the passenger railway car through the outlet window of the device.

### Example 14.

The ACS is an air conditioning system (device) equipped with an ultraviolet air treatment unit 8 (UAT unit), mounted downstream of a mixing chamber 3 and upstream of an evaporation unit 5. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to adjacent parts of the main air duct 4 of the device, preferably by a flange connection, and is mounted between the mixing chamber and the main air duct in accordance with the general configuration of an ACS.

On the air inlet window 10 of the air-duct housing 9 of the unit 8, a means for protecting against UV radiation is mounted, in the form of a protective profiled grille 15. The protective grille 15 is secured on the air outlet window 10, and its surface is provided with a photocatalytic coating based on titanium dioxide (TiO₂), which absorbs UV radiation and additionally improves the effectiveness of the disinfection and cleaning of the air due to the photocatalysis.

As a result, the sensitive parts and components (made from materials that are resistant to UV radiation) of the system, located in the direction of the air inlet window 10 into the UAT unit 8, wherein the emission of UV radiation outside of the UAT unit 8 in this direction is prevented.

In addition, due to the fact that a coating that reflects UV radiation is applied to the internal surface of the air-duct housing 9 of the UAT unit 8, the conditions are created for the propagation of the UV radiation beyond the UAT unit 8 towards the air outlet window 11, and the treatment will be extended to the surfaces located behind the UAT unit 8 in the direction of the evaporation unit, wherein the air treatment zone is also extended beyond the confines of the unit 8, and the duration of the air treatment is increased accordingly, which will have a positive impact on the results of the disinfection.

The device works as follows. With the inlet fan 6 switched on, air is pumped along the entire length of the air duct of the air conditioning system, and both streams of the external and recirculated air reach the mixing chamber 3 from the air intake units 2 and 1. From the mixing chamber 3, the air enters the UAT unit 8, installed between the mixing chamber and the evaporation unit, where the air flow is irradiated by the ultraviolet amalgam lamp 13, or lamps if there are more than one, and the physical construction of the unit with the protective grille 15 coated with titanium dioxide and mounted on the inlet window 10 of the unit 8, in combination with the coating that reflects UV radiation applied to the inside surface of the air-duct housing 9 of the unit 8, does not allow the radiation to be emitted in the direction of the mixing chamber 3 and at the same time, allows the treatment zone for the air and the surfaces located outside the UAT unit 8 to be extended in the direction of the air flow, increasing the likelihood of destroying the living micro-organisms in the air stream. Further along the main air duct 4, the airflow passes through the evaporation unit 5 and the inlet fan 6 to reach the forced-draft air duct 7, from where the cleaned and decontaminated air is fed into the passenger railway car through the outlet window of the device.

### Example 15.

The ACS is an air conditioning system (device) equipped with an ultraviolet air treatment unit 8 (UAT unit), mounted downstream of a mixing chamber 3 and upstream of an evaporation unit 5. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to adjacent parts of the main air duct 4 of the device, preferably by a flange connection, and is mounted between the mixing chamber and the main air duct in accordance with the general configuration of an ACS.

On the outlet window 11 of the air-duct housing 9 of the unit 8, a means for protecting against UV radiation is mounted, in the form of a protective profiled grille 15. The protective grille 15 is secured on the air outlet window 11, and its surface is provided with a photocatalytic coating based on titanium dioxide (Ti02), which absorbs UV radiation and additionally improves the effectiveness of the disinfection and cleaning of the air due to the photocatalysis.

As a result, the sensitive parts and components (made from materials that are resistant to UV radiation) of the system, located in the direction of the air inlet window 11 into the UAT unit 8, are protected from UV radiation, wherein the emission of radiation outside of the UAT unit 8 in this direction is prevented.

In addition, due to the fact that a coating that reflects UV radiation is applied to the internal surface of the air-duct housing 9 of the UAT unit 8, the conditions are created for the propagation of the UV radiation beyond the confines of the UAT unit 8, and the treatment will be extended to the surfaces located upstream of the UAT unit 8 in the direction of the mixing chamber 3, wherein the air treatment zone is also extended beyond the confines of the unit 8, and the duration of the air treatment is increased accordingly, which will have a positive impact on the results of the disinfection.

The device works as follows. With the inlet fan 6 switched on, air is pumped along the entire length of the air duct of the air conditioning system, and both streams of the external and recirculated air reach the mixing chamber 3 from the air intake units 2 and 1. From the mixing chamber 3, the air enters the UAT unit 8, installed between the mixing chamber 3 and the evaporation unit 5, where the air flow is irradiated by the ultraviolet amalgam lamp 13, or lamps if there are more than one, and the physical construction of the UAT unit 8 with the protective grille 15 coated with titanium dioxide and mounted on the outlet window 10 of the unit 8, in combination with the coating that reflects UV radiation which is applied to the internal surface of the air-duct housing 9 of the unit 8, does not allow the radiation to be emitted in the direction of the evaporation unit 5, while at the same time allowing the treatment zone for the air and the surfaces located outside the UAT unit 8 to be extended in the direction of the mixing chamber 3, increasing the likelihood of destroying the living micro-organisms in the air stream and on the adjoining surfaces. Further along the main air duct 4, the airflow passes through the evaporation unit 5 and the inlet fan 6 to reach the forced-draft air duct 7, from where the cleaned and decontaminated air is fed into the passenger railway car through the outlet window of the system.

### Example 16.

The ACS is an air conditioning system (device) equipped with an ultraviolet air treatment unit 8 (UAT unit), mounted downstream of the unit and upstream of the inlet fan. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to the adjacent parts of the main air duct 4 preferably by a flange connection, or is mounted between the evaporation unit and the inlet fan in accordance with the general configuration of an ACS.

On the air inlet 10 and air outlet 11 windows of the air-duct housing 9 of the unit 8, means for protection against UV radiation are mounted, in the form of protective profiled grilles 15. Protective grilles 15 are installed on both windows of the UAT unit 8, i.e. on the air inlet window 10 from the direction of the evaporation unit 5 and on the outlet window 11 for the treated air in the direction of the inlet fan 6. The surfaces of the protective grilles 15 are each provided with a photocatalytic coating based on titanium dioxide (TiO₂) that absorbs UV radiation and additionally improves the effectiveness of the disinfection and cleaning of the air due to photocatalysis.

In addition, in a particular embodiment, the titanium dioxide coating can be applied on the internal surface of the air-duct housing 9 of the UAT unit 8, which means it is possible to provide significant reduction in the UV radiation emission in both directions from the unit 8 due to the dual protection: the UV radiation is absorbed by the coating of the internal surface of the air-duct housing 9 and by the protective grilles 15 on the air inlet and outlet windows 10 and 11. This prevents the emission of UV radiation beyond the confines of the UAT unit 8, protecting the assemblies and components of the device from the damaging effects of the UV radiation. This is necessary because materials which are not resistant to the action of UV radiation may be used in the construction of the device, such as galvanized steel and porous rubber, which is used as a sealing material, etc.

The device works as follows. With the inlet fan 6 switched on, air is pumped along the entire length of the air duct of the air conditioning system, and both streams of the external and recirculated air reach the mixing chamber 3 from the air intake units 2 and 1. Then, from the mixing chamber 3 the air flow passes into the evaporation unit 5, mounted downstream of the mixing chamber 3 and then into the UAT unit 8.

In the UAT unit 8 the air is continuously treated by the UV radiation from the lamp 13, then the decontaminated air exits the unit 8 to reach the inlet fan 6, and later, enters the forced-draft air duct 7, from where the cleaned and decontaminated air is fed into the passenger railway car through the outlet window.

In the UAT unit 8 the air flow is irradiated by the ultraviolet amalgam lamp 13, or lamps if there are more than one, and the physical construction of the unit with the two protective grilles 15, coated with titanium dioxide and mounted on the inlet 10 and outlet windows 11 of the unit 8, in combination with the same coating on the inside of the air-duct housing 9 of the unit 8 (which is applied in particular embodiments of the unit 8), safely contain the UV radiation within the UAT unit 8. As a result, the main assemblies and components of the device are protected from the UV radiation, since the emission of radiation outside of the unit 8 and thus any effect on the people present in the railway car compartment is prevented.

### Example 17.

The ACS is an air conditioning system (device) equipped with an ultraviolet air treatment unit 8 (UAT unit), mounted downstream of the unit and upstream of the inlet fan. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to the adjacent parts of the main air duct 4, preferably by a flange connection, or is mounted between the evaporation unit and the inlet fan in accordance with the general configuration of an ACS.

On the inlet window 10 of the air-duct housing 9 of the unit 8, means for protection against UV radiation are mounted, in the form of a protective profiled grille 15. The protective grille 15 is fixed on the air inlet window 10 from the direction of the evaporation unit 5. The surfaces of the protective grilles 15 are each provided with a photocatalytic coating based on titanium dioxide (TiO₂), which absorbs UV radiation and additionally improves the effectiveness of the disinfection and cleaning of the air due to photocatalysis.

In addition, in a particular embodiment, the titanium dioxide (TiO₂) coating can be applied on the internal surface of the air-duct housing 9 of the UAT unit 8, which means it is possible to provide a significant reduction in the UV radiation emission in the direction of the passenger compartment in both directions away from the unit 8 due to the dual protection: the UV radiation is absorbed by the coating of the internal surface of the air-duct housing 9 and of the protective grille 15 on the air inlet window 10; this prevents the emission of UV radiation outside the confines of the UAT unit 8 and protects the assemblies and components of the system from the damaging effect of the UV radiation. Application of a coating that absorbs UV radiation on the internal surface of the air-duct housing 9, allows the radiation output in the direction of the inlet fan to be limited. This is necessary because materials which are not resistant to the action of UV radiation may be used in the construction of the device, such as galvanized steel, porous rubber, which is used as a sealing material for maintenance hatches etc.

The device works as follows. With the inlet fan 6 switched on, air is pumped along the entire length of the air duct of the air conditioning system, and both streams of the external and recirculated air reach the mixing chamber 3 from the air intake units 2 and 1. Then, from the mixing chamber 3 the air flow passes into the evaporation unit 5, mounted downstream of the mixing chamber 3 and then into the UAT unit 8.

In the UAT unit 8 the air is continuously treated by the UV radiation from the lamp 13, then the decontaminated air, having passed from the UAT unit 8 to the inlet fan 6, continues on to the forced-draft air duct 7 downstream of the fan 6, from where the cleaned and decontaminated air is fed into the passenger railway car through the outlet window.

In the UAT unit 8, the air flow is irradiated by the ultraviolet amalgam lamp 13, or lamps if there are more than one, and the physical construction of the unit with the protective grille 15 coated with titanium dioxide and mounted on the inlet window 10 of the UAT unit 8, in combination with the same coating on the inside of the air-duct housing 9 of the unit 8 (which is applied in particular embodiments of the unit 8), safely contain the UV radiation within the UAT unit 8. As a result, the main assemblies and components of the device are protected from the UV radiation, since the emission of radiation outside of the unit 8 and thus any effect on the people present in the railway car compartment is prevented.

### Example 18.

The ACS is an air conditioning system (device) equipped with an ultraviolet air treatment unit 8 (UAT unit), mounted downstream of the unit and upstream of the inlet fan. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to the adjacent parts of the main air duct 4 preferably by a flange connection, or is mounted between the evaporation unit and the inlet fan in accordance with the general configuration of an ACS.

On the outlet window 11 of the air-duct housing 9 of the unit 8, a means for protecting against UV radiation is mounted, in the form of a protective profiled grille 15. The protective grille 15 is fixed on the air inlet window 11 from the direction of the inlet fan 6. The surface of the protective grille 15 is provided with a photocatalytic coating based on titanium dioxide (TiO₂), which absorbs UV radiation and additionally improves the effectiveness of the disinfection and cleaning of the air due to photocatalysis.

In addition, in a particular embodiment the coating based on titanium dioxide can be applied to the internal surface of the air-duct housing 9 of the UAT unit 8, which means it is possible to provide a significant reduction in the UV radiation emission in both directions from the unit 8, wherein in the direction of the inlet fan the emission of radiation is blocked by a dual protection: the UV radiation is absorbed by the coating on the internal surface of the air-duct housing 9 and by the protective grille 15 on the air outlet window 11, and in the direction of the evaporation unit the radiation emission is attenuated due to the coating on the internal surface of the UAT unit 8, since the coating on the internal surface of the air-duct housing 9, which absorbs UV radiation, enables the radiation output to be limited to the side where the protective grille is absent - in the direction of the evaporation unit 5. This is necessary because materials which are not resistant to the action of UV radiation may be used in the construction of the ACS, such as galvanized steel, porous rubber, which is used as a sealing material etc.

The device works as follows. With the inlet fan 6 switched on, air is pumped along the entire length of the air duct of the air conditioning system, and both streams of the external and recirculated air reach the mixing chamber 3 from the air intake units 2 and 1. Then, from the mixing chamber 3 the air flow passes into the evaporation unit 5, mounted downstream of the mixing chamber 3 and then into the UAT unit 8.

In the UAT unit 8 the air is continuously treated by the UV radiation from the lamp 13, then the decontaminated air passes through the inlet fan 6 and later enters the forced-draft air duct 7, from where the cleaned and decontaminated air is fed into the passenger railway car through the outlet window.

In the UAT unit 8, the air flow is irradiated by the ultraviolet amalgam lamp 13, or lamps if there are more than one, and the physical construction of the unit with the protective grille 15 coated with titanium dioxide and mounted on the outlet window 11 of the UAT unit 8, in combination with the same coating on the inside of the air-duct housing 9 of the unit 8 (which is applied in particular embodiments of the unit 8), safely contain the UV radiation within the UAT unit 8. As a result, the main assemblies and components of the device are protected from the UV radiation, since the emission of radiation outside of the unit 8 and thus any effect on the people present in the railway car compartment is prevented.

### Example 19.

The ACS is an air conditioning system (device) equipped with an ultraviolet air treatment unit 8 (UAT unit), mounted downstream of the unit and upstream of the inlet fan. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to the adjacent parts of the main air duct 4, preferably by a flange connection, and is installed in accordance with the general configuration of an ACS. The protective grilles in the construction of the UAT unit 8 are omitted.

In a particular embodiment, the titanium dioxide (TiO₂) coating can be applied to the internal surface of the air-duct housing 9 of the UAT unit 8, which results in a significant reduction of the UV radiation emission beyond the confines of the UAT unit 8 being achieved, in addition to improved effectiveness of the disinfection and cleaning of the air due to the photocatalysis.

If a coating based on TiO₂ is applied to the internal surface of the air-duct housing of the unit 8, this will prevent the emission of UV radiation in the direction of the evaporation unit 5 and the inlet fan 6, protecting the assemblies and components of the ACS from the destructive effect of the UV radiation. This is necessary because materials which are not resistant to the action of UV radiation may also be used in the construction of the air conditioning system, such as galvanized steel, porous rubber, which is used as a sealing material etc.

If the evaporation unit 5 and the inlet fan 6 are implemented from materials that are resistant to UV radiation, then no TiO₂-based coating needs to be applied to the internal surface of the air-duct housing of the unit 8. In this case, the UV radiation will propagate beyond the confines of the unit 8, and the treatment will be applied to the surfaces located both upstream of the unit 8 and downstream of the unit 8, but the range and duration of the air treatment will also extend beyond the confines of the UAT unit 8, which will have a positive impact on the disinfection results.

The device works as follows. With the inlet fan 6 switched on, air is pumped along the entire length of the air duct of the air conditioning system, and both streams of the external and recirculated air from the air intake units 2 and 1 reach the mixing chamber 3, and then the air stream is directed into the evaporation unit 5, after which it reaches the UAT unit 8. In the UAT unit installed between the evaporation unit 5 and the inlet fan, the air flow is irradiated by the ultraviolet amalgam lamp 13, or lamps if there are more than one, and the physical construction of the unit with a coating of TiO₂ on the inside of the air-duct housing 9 of the unit 8 (which is applied in particular embodiments of the unit 8), safely confines the UV radiation within the UAT unit 8. As a result, the assemblies and components of the air conditioning system that are sensitive to the UV radiation are protected. If there is no coating on the inside of the air-duct housing 9 of the unit 8, the UV radiation will be released outside the confines of the unit 8 in the direction opposite to the air flow, towards the evaporation unit 5 and in the opposite direction, towards the inlet fan, which allows the surfaces of the air conditioning system located outside of the unit 8 to be treated, and also the distance and duration over which the air is treated to be increased, i.e. the air treatment takes place not only in the unit 8, but also before entering and after exiting the unit 8. Further along the main air duct 4 from the UAT unit 8, the airflow passes through the inlet fan 6 to enter the forced-draft air duct 7, from where the cleaned and decontaminated air is fed into the passenger railway car through the outlet window of the device.

### Example 20.

The ACS is an air conditioning system (device) equipped with an ultraviolet air treatment unit 8 (UAT unit), mounted downstream of the unit and upstream of the inlet fan. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to the adjacent parts of the main air duct 4, preferably by a flange connection, and is installed in accordance with the general configuration of an ACS. The protective grilles in the construction of the UAT unit 8 are omitted.

The coating reflecting the UV radiation, which is applied to the internal surface of the air-duct housing 9 of the UAT unit 8, which additionally enables the efficiency of the disinfection and cleaning of the air to be increased, due to the fact that the UV radiation will propagate beyond the confines of the UAT unit 8, and the treatment will be extended to surfaces located both upstream of the unit 8 and downstream of the unit 8. The treatment area is also extended beyond the confines of the unit 8, and the duration of the air treatment is increased, that will have a positive impact on the disinfection results.

In the construction of the air conditioning system, in areas adjacent to the UAT unit, materials resistant to UV radiation must be used.

The device works as follows. With the inlet fan 6 switched on, air is pumped along the entire length of the air duct of the air conditioning system, and both streams of the external and recirculated air reach the mixing chamber 3 from the air intake units 2 and 1. From the mixing chamber the air flow passes into the evaporation unit 5 and then onto the UAT unit 8. In the UAT unit installed downstream of the evaporation unit and upstream of the inlet fan, the air flow is irradiated by an ultraviolet amalgam lamp 13, or lamps if there are more than one, and the embodiment of the unit with a coating reflecting UV radiation on the inside of the air-duct housing 9 of the unit 8 allows the radiation to be emitted beyond the confines of the unit 8 in the direction of the air flow and in the opposite direction, which causes treatment of the surfaces located outside the unit 8, as well as the air supplied into the unit 8 and exiting from it. Then, along the main air duct 4 from the UAT unit, the airflow passes through the inlet fan 6 to enter the forced-draft air duct 7, from where the cleaned and decontaminated air is fed into the passenger railway car through the outlet window of the device.

### Example 21.

The ACS is an air conditioning system (device) equipped with an ultraviolet air treatment unit 8 (UAT unit), mounted downstream of the unit and upstream of the inlet fan. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to the adjacent parts of the main air duct 4, preferably by a flange connection, and is installed in accordance with the general configuration of an ACS.

On the inlet window 10 of the air-duct housing 9 of the unit 8, means for protection against UV radiation are mounted, in the form of a protective profiled grille 15. The protective grille 15 is mounted on the air inlet window 10 of the UAT unit 8, and its surface is provided with a photocatalytic coating based on titanium dioxide (TiO₂), which absorbs UV radiation and additionally improves the effectiveness of the disinfection and cleaning of the air due to the photocatalysis.

As a result, the sensitive assemblies and components (made from materials that are not resistant to UV radiation) of the system, located in the direction of the air inlet window 10 into the UAT unit 8, wherein the emission of radiation outside of the UAT unit 8 in this direction is almost eliminated.

In addition, due to the fact that a coating that reflects UV radiation is applied to the internal surface of the air-duct housing 9 of the UAT unit 8, the conditions are created for the propagation of the UV radiation beyond the confines of the UAT unit 8 towards the place where the protective grill is absent, and the treatment will be applied to the surfaces located downstream of the UAT unit 8 in the direction of the inlet fan 6. The air treatment area is also extended beyond the confines of the unit 8, and the duration of the air treatment is correspondingly increased, which will have a positive impact on the results of the disinfection.

The device works as follows. With the inlet fan 6 switched on, air is pumped along the entire length of the air duct of the air conditioning system, and both streams of the external and recirculated air reach the mixing chamber 3 from the air intake units 2 and 1. From the mixing chamber, air enters the evaporation unit and then passes to the UAT unit 8, mounted downstream of the evaporation unit and upstream of the inlet fan. In the UAT unit 8, the air flow is irradiated by the ultraviolet amalgam lamp 13, or lamps if there are more than one, and the physical construction of the unit with the protective grille 15 coated with titanium dioxide and mounted on the inlet window 10 of the unit 8, in combination with the coating that reflects UV radiation applied to the inside surface of the air-duct housing 9 of the unit 8, does not allow radiation to be emitted in the direction of the evaporation unit 5 and at the same time, allows the treatment zone for the air and the surfaces located outside the UAT unit 8 to be extended in the direction of the air flow, increasing the likelihood of destroying the living micro-organisms in the air stream and on the adjoining surfaces. Next, the airflow passes through the inlet fan 6 to arrive at the forced-draft air duct 7, from where the cleaned and decontaminated air is fed into the passenger railway car through the outlet window of the device.

### Example 22.

The ACS is an air conditioning system (device) equipped with an ultraviolet air treatment unit 8 (UAT unit), mounted downstream of the inlet fan. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected using mounting elements of the inlet fan to the forced-draft air duct 7, preferably by a flange connection, and is installed in accordance with the general configuration of an ACS.

On the air inlet window 10 and air outlet window 11 of the air-duct housing 9 of the unit 8, means for protection against UV radiation are mounted, in the form of protective profiled grilles 15. The protective grilles 15 are installed on both windows of the UAT unit 8, i.e. on the air inlet window 10 away from the inlet fan 6 and on the air outlet window 11 into the forced-draft air duct 7, and their surface is provided with a photocatalytic coating based on titanium dioxide (TiO₂), which absorbs UV radiation and additionally improves the effectiveness of the disinfection and cleaning of the air due to photocatalysis.

In a particular embodiment of a utility model, the titanium dioxide (TiO₂) coating can be additionally applied to the internal surface of the air-duct housing 9 of the UAT unit 8, which ensures a significant reduction of the UV radiation emission in both directions in relation to the UAT unit 8.

The installation of the protective grille 15 on the air inlet window 10 prevents the emission of UV radiation outside the confines of the device towards the inlet fan and prevents any exposure on its surface, which is important if the design of the fan inlet uses materials which are not resistant to UV radiation. The protective grille 15 on the air outlet window 11 prevents UV radiation from reaching the output forced-draft air duct 7, from which, reflecting off the internal surfaces of the air duct 7, the UV radiation would have been able to reach the passenger compartment of the car, which is inadmissible.

The device works as follows. With the inlet fan 6 switched on, air is pumped along the entire length of the air duct of the air conditioning system, and both streams of the external and recirculated air reach the mixing chamber 3 from the air intake units 2 and 1. The air then flows along the main air duct 4 through the evaporation unit 5 and the inlet fan 6, and arrives at the UAT unit 8. From the UAT unit 8 the cleaned and disinfected air enters the forced-draft air duct 7 and is fed through the outlet window into the passenger railway compartment.

In the UAT unit 8 the air flow is irradiated by the ultraviolet amalgam lamp 13, or lamps if there are more than one, and the physical construction of the unit with the two protective grilles 15, coated with titanium dioxide and mounted on the inlet 10 and outlet windows 11 of the unit 8, in combination with the same coating on the inside of the air-duct housing 9 of the unit 8 (which is applied in particular embodiments of the unit 8), safely confine the UV radiation within the UAT unit 8. As a result, the main assemblies and components of the ACS are protected from UV radiation and any effect of it on the people present in the railway car compartment is eliminated.

### Example 23.

The ACS is an air conditioning system (device) equipped with an ultraviolet air treatment unit 8 (UAT unit), mounted downstream of the inlet fan. Lamp assemblies 12 with electrical connectors for one, two or more sources of germicidal UV radiation - in the form of amalgam lamps 13, primarily having a U-shaped form - are secured to the wall of the air-duct housing of the UAT unit. The UAT unit is connected to mounting elements of the inlet fan 6 and to the forced-draft air duct 7, preferably by a flange connection, and is installed in accordance with the general configuration of an ACS.

The UAT unit 8 has flanges 14 for installing the UAT unit 8 in the forced-draft air duct 7 or for securing it to the forced-draft air duct 7 and the stationary elements of the inlet fan 6, i.e. it can be mounted in accordance with the configuration of an air conditioning system.

On the air inlet window 10 of the air-duct housing 9 of the unit 8, a means for protecting against UV radiation is mounted, in the form of a protective profiled grille 15, on the surface of which is applied a photocatalytic coating based on titanium dioxide (TiO₂), which absorbs UV radiation and further improves the effectiveness of the disinfection and cleaning of the air due to the photocatalysis.

In a particular embodiment of a utility model, the titanium dioxide (TiO₂) coating can be additionally applied to the internal surface of the air-duct housing 9 of the UAT unit 8, which means it is possible to provide a significant reduction in the UV radiation emission in the direction of the inlet fan due to the dual protection: the UV radiation is absorbed by the coating of the internal surface of the air-duct housing 9 and of the protective grille 15 on the air inlet window 10, which prevents it from acting on the surfaces of the inlet fan, which is important if materials are used in the construction of the inlet fan that are not resistant to UV radiation, In addition, the coating on the internal surface of the air-duct housing 9 reduces the emission of UV radiation out of the device through the air outlet window 11 in the forced-draft air outlet duct 7, from which, reflecting off the internal surfaces of the air duct 7, the UV radiation would have been able to reach the passenger compartment of the car, which is inadmissible.

The device works as follows. With the inlet fan 6 switched on, air is pumped along the entire length of the air duct of the air conditioning system, and both streams of the external and recirculated air reach the mixing chamber 3 from the air intake units 2 and 1. The air then flows along the main air duct 4 through the evaporation unit 5 and the inlet fan 6, and arrives at the UAT unit 8. From the UAT unit 8, the cleaned and disinfected air enters the forced-draft air duct 7 and is fed through the outlet window into the passenger railway compartment.

In the UAT unit 8, the air flow is irradiated by the ultraviolet amalgam lamp 13, or lamps if there are more than one, and the physical construction of the unit with the protective grille 15 coated with titanium dioxide and mounted on the inlet window 10 of the unit 8, in combination with the same coating on the inside of the air-duct housing 9 of the unit 8 (which is applied in particular embodiments of the unit 8), safely contain the UV radiation within the UAT unit 8. As a result, the main assemblies and components of the air conditioning system are protected from UV radiation, while at the same time reducing the possibility of radiation emission outside of the confines of the unit 8 and of any effect it may have on the people present in the railway car compartment.

Thus, due to the plurality of assembly variants of the UAT unit, optimum dimensional characteristics of the UAT unit can be achieved and the UAT unit can be arranged at virtually any position in the ACS, and as a result of the above described design of the UAT unit, the efficiency of the air disinfection is increased while extending the service life and fitness for use of the device (protection of the assemblies and parts from exposure to ultraviolet light), as well as ensuring reliable protection for passengers and staff from pathogenic micro-organisms and the effects of UV radiation. In addition, a reduced size of the air conditioning device and improved air treatment performance are provided (recirculation ratio of 2-27, which provides sufficient reduction of the growth of the colony of micro-organisms).

## Claims

1. A railway car air conditioning system comprising an inlet recirculation air duct, an inlet air duct for external air, subsequently arranged a chamber for mixing the external and recirculated air, a main air duct in which an evaporation unit and inlet fan are arranged, and an output forced-draft air duct feeding the processed air into the passenger railway car, also comprising a source of ultraviolet radiation, in which the system is equipped with means for protection against the emission of ultraviolet radiation, and a coating is applied onto the components of the system that absorbs ultraviolet radiation, **characterized in that** the system is equipped with a UV air treatment unit, consisting of an air-duct housing in which a source of ultraviolet radiation is arranged, the source being at least one amalgam lamp, wherein the unit is installed in at least one of the following places: upstream of the mixing chamber in the inlet recirculation air duct, and/or in the mixing chamber, and/or downstream of the mixing chamber and upstream of the evaporation unit, and/or downstream of the evaporation unit and upstream of the inlet fan, and/or downstream of the inlet fan in the output forced-draft air duct, wherein, in the air-duct housing of the unit, windows for air inlet and outlet are closed off by protective grilles, onto which a titanium dioxide coating that absorbs ultraviolet radiation is applied, wherein the evaporation unit is arranged upstream of the inlet fan.

2. The system as claimed in claim 1, **characterized in that** lamp clusters with electrical connectors for one, two or more sources of germicidal ultraviolet radiation are secured to the wall of the air-duct housing of the UV air treatment unit, wherein U-shaped amalgam lamps are used as the sources of germicidal ultraviolet radiation.

3. The system as claimed in claim 2, **characterized in that** if two or more U-shaped amalgam lamps are used the lamp clusters are mounted in pairs one under another or along the axis of the air-duct housing of the unit on a single line, or with a height offset to allow the arrangement of the amalgam lamps one under the other or one behind the other.

4. The system as claimed in claim 1, **characterized in that**, on the inside surface of the air-duct housing, a coating that absorbs ultraviolet radiation, namely titanium dioxide, is applied.

5. An air conditioning system for a railway car compartment, comprising an inlet recirculation air duct, an inlet air duct for external air, subsequently arranged a chamber for mixing the external and recirculated air, a main air duct in which an evaporation unit and inlet fan are arranged, and an output forced-draft air duct feeding the processed air into the compartment, also comprising a source of ultraviolet radiation, wherein the system is equipped with means for protection against the emission of ultraviolet radiation, and a coating that absorbs ultraviolet radiation and/or a coating that reflects ultraviolet radiation is applied onto the components of the system, **characterized in that** the system is equipped with a UV air treatment unit consisting of an air-duct housing in which a source of ultraviolet radiation is arranged, the source being at least one amalgam lamp, wherein the unit is installed in at least one of the following places: upstream of the mixing chamber in the inlet recirculation air duct, and/or in the mixing chamber, and/or downstream of the mixing chamber and upstream of the evaporation unit, and/or downstream of the evaporation unit and upstream of the inlet fan, and/or downstream of the inlet fan in the output forced-draft air duct, wherein, in the air-duct housing of the unit, an inlet window or an air outlet window for the air is closed off by a protective grille, onto which a titanium dioxide coating that absorbs ultraviolet radiation is applied, wherein the evaporation unit is arranged upstream of the inlet fan.

6. The system as claimed in claim 5, **characterized in that** lamp clusters with electrical connectors for one, two or more sources of germicidal ultraviolet radiation are secured to the wall of the air-duct housing of the UV air treatment unit, wherein U-shaped amalgam lamps are used as the sources of germicidal ultraviolet radiation.

7. The system as claimed in claim 5, **characterized in that** when two or more U-shaped amalgam lamps are used the lamp clusters are mounted in pairs one under another or along the axis of the air-duct housing of the unit on a single line, or with a height offset to allow the arrangement of the amalgam lamps one under the other or one behind the other.

8. The system as claimed in claim 5, **characterized in that**, if the ultraviolet air treatment unit is arranged upstream of the mixing chamber in the recirculation air duct, the protective grille with a coating of titanium dioxide is arranged on the air inlet window, wherein the coating of titanium dioxide is applied onto the internal surface of the air-duct housing of the UV air treatment unit.

9. The system as claimed in claim 5, **characterized in that**, if the UV air treatment unit is arranged in the mixing chamber, the protective grille with the titanium dioxide coating is arranged on the air inlet window into the unit or the protective grille with a titanium dioxide coating is arranged on the air outlet window.

10. The system as claimed in claim 9, **characterized in that**, if the UV air treatment unit is arranged in the mixing chamber, the titanium dioxide coating is applied to the internal surface of the air-duct housing of the UV air treatment unit.

11. The system as claimed in claim 5, **characterized in that**, if the UV air treatment unit is arranged in the mixing chamber, the protective grille with the titanium dioxide coating is arranged on the air inlet window into the UV air treatment unit.

12. The system as claimed in claim 11, **characterized in that**, if the UV air treatment unit is arranged in the mixing chamber, a coating that reflects ultraviolet radiation is applied to the internal surface of the ultraviolet air treatment unit.

13. The system as claimed in claim 5, **characterized in that**, if the UV air treatment unit is installed downstream of the mixing chamber and upstream of the evaporation unit, the protective grille with the titanium dioxide coating is arranged on the air inlet window into the unit or the protective grille with the titanium dioxide coating is arranged on the air outlet window.

14. The system as claimed in claim 13, **characterized in that**, if the UV air treatment unit is arranged downstream of the mixing chamber and upstream of the evaporation unit, the titanium dioxide coating is applied to the internal surface of the air-duct housing of the UV air treatment unit.

15. The system as claimed in claim 5, **characterized in that**, if the UV air treatment unit is arranged downstream of the mixing chamber and upstream of the evaporation unit, the protective grille with the titanium dioxide coating is arranged on the air inlet window into the UV air treatment unit or on the air outlet window of the unit.

16. The system as claimed in claim 15, **characterized in that**, if the UV air treatment unit is arranged downstream of the mixing chamber and upstream of the evaporation unit, a coating that reflects ultraviolet radiation is applied onto the internal surface of the UV air treatment unit.

17. The system as claimed in claim 5, **characterized in that**, if the UV air treatment unit is installed downstream of the evaporation unit and upstream of the inlet fan, the protective grille with the titanium dioxide coating is arranged on the air inlet window into the unit or the protective grille with the titanium dioxide coating is arranged on the air outlet window.

18. The system as claimed in claim 17, **characterized in that**, if the UV air treatment unit is arranged downstream of the evaporation unit and upstream of the inlet fan, the titanium dioxide coating is applied onto the internal surface of the air-duct housing of the UV air treatment unit.

19. The system as claimed in claim 5, **characterized in that**, if the UV air treatment unit is installed downstream of the evaporation unit and upstream of the inlet fan, the protective grille with the titanium dioxide coating is arranged on the air inlet window into the UV air treatment unit, wherein a coating that reflects ultraviolet radiation is applied onto the internal surface of the UV air treatment unit.

20. The system as claimed in claim 5, **characterized in that**, if the UV air treatment unit is arranged downstream of the inlet fan in the forced-draft air duct, the protective grille with the titanium dioxide coating is arranged on the air inlet window into the unit.

21. The system as claimed in claim 20, **characterized in that**, if the UV air treatment unit is arranged downstream of the inlet fan, the titanium dioxide coating is applied onto the internal surface of the air-duct housing of the UV air treatment unit.

22. An air conditioning system for a railway car compartment, comprising an inlet recirculation air duct, an inlet air duct for external air, subsequently arranged a chamber for mixing the external and recirculated air, a main air duct in which an evaporation unit and inlet fan are arranged, and an output forced-draft air duct feeding the processed air into the compartment, also comprising a source of ultraviolet radiation, wherein the system is equipped with means for protection against the emission of ultraviolet radiation, and a coating that absorbs ultraviolet radiation and/or a coating that reflects ultraviolet radiation is applied onto the components of the system, **characterized in that** the system is equipped with a UV air treatment unit consisting of an air-duct housing in which a source of ultraviolet radiation is arranged, the source being at least one amalgam lamp, wherein the unit is installed in at least one of the following places: in the mixing chamber, and/or downstream of the mixing chamber and upstream of the evaporation unit, and/or downstream of the evaporation unit and upstream of the inlet fan, wherein the evaporation unit is installed upstream of the inlet fan.

23. The system as claimed in claim 22, **characterized in that** lamp clusters with electrical connectors for one, two or more sources of germicidal ultraviolet radiation are secured to the wall of the air-duct housing of the UV air treatment unit, wherein U-shaped amalgam lamps are used as the sources of germicidal ultraviolet radiation.

24. The system as claimed in claim 22, **characterized in that** when two or more U-shaped amalgam lamps are used the lamp clusters are mounted in pairs one under another or along the axis of the air-duct housing of the unit on a single line, or with a height offset to allow the arrangement of the amalgam lamps one under the other or one behind the other.

25. The system as claimed in claim 22, **characterized in that**, if the UV air treatment unit is arranged in the mixing chamber, a titanium dioxide coating is applied onto the internal surface of the air-duct housing of the UV air treatment unit.

26. The system as claimed in claim 22, **characterized in that**, when the UV air treatment unit is arranged in the mixing chamber, the coating that reflects ultraviolet radiation is applied onto the internal surface of the ultraviolet air treatment unit.

27. The system as claimed in claim 22, **characterized in that**, if the UV air treatment unit is arranged downstream of the mixing chamber and upstream of the evaporation unit, a titanium dioxide coating is applied onto the internal surface of the air-duct housing of the UV air treatment unit.

28. The system as claimed in claim 22, **characterized in that**, when the UV air treatment unit is arranged downstream of the mixing chamber and upstream of the evaporation unit, the coating that reflects ultraviolet radiation is applied onto the internal surface of the UV air treatment unit.

29. The system as claimed in claim 22, **characterized in that**, if the UV air treatment unit is arranged downstream of the evaporation unit and upstream of the inlet fan, a titanium dioxide coating is applied onto the internal surface of the air-duct housing of the UV air treatment unit.

30. The system as claimed in claim 22, **characterized in that**, if the UV air treatment unit is arranged downstream of the evaporation unit and upstream of the inlet fan, the coating that reflects ultraviolet radiation is applied onto the internal surface of the UV air treatment unit.
